# EUROPEAN PATENT SPECIFICATION

(11) **EP 3 706 799 B1**
(45) Date of publication and mention of the grant of the patent: **12.03.2025**
(21) Application number: 18877124.0
(22) Date of filing: 08.11.2018
(51) Int. Cl.: A61K 38/17, A61K 45/06, A61P 9/10, A61P 9/00

(54) **PEPTIDE FOR PREVENTING, MODULATING AND/OR REDUCING CARDIOVASCULAR DISEASE**
PEPTID ZUR VORBEUGUNG, MODULATION UND / ODER VERMINDERUNG KARDIOVASKULÄRER ERKRANKUNGEN
PEPTIDE POUR LA PRÉVENTION, LA MODULATION ET/OU LA RÉDUCTION D'UNE MALADIE CARDIOVASCULAIRE

(30) Priority: 10.11.2017 US 201762584595 P; 10.05.2018 US 201862669918 P
(43) Date of publication of application: 16.09.2020
(73) Proprietor: OP-T LLC, Aurora, CO 80045 (US)
(72) Inventor: WAGNER, David Hal, Jr., Denver, Colorado 80220 (US); YUSSMAN, Martin Glenn, Denver, Colorado 80128 (US); HENRY, Charles W., Denver, Colorado 80206 (US)
(74) Representative: ZBM Patents - Zea, Barlocci & Markvardsen
(86) International application number: PCT/US2018/059810
(87) International publication number: WO 2019/094581

(56) References cited:
- WO-A1-99/11263
- US-A1- 2004 072 750
- US-A1- 2013 236 495
- US-A1- 2014 135 684
- US-B2- 7 189 518
- STEIN COLLEEN S. ET AL: "Long-term reversal of hypercholesterolemia in low density lipoprotein receptor (LDLR)-deficient mice by adenovirus-mediated LDLR gene transfer combined with CD154 blockade", THE JOURNAL OF GENE MEDICINE, vol. 2, no. 1, 1 January 2000 (2000-01-01), US, pages 41 - 51, XP055824677, ISSN: 1099-498X, DOI: 10.1002/(SICI)1521-2254(200001/02)2:1<41::AID-JGM79>3.0.CO;2-P
- LUTGENS ESTHER ET AL: "Requirement for CD154 in the progression of atherosclerosis", NATURE MEDICINE, vol. 5, no. 11, 1 November 1999 (1999-11-01), New York, pages 1313 - 1316, XP055824674, ISSN: 1078-8956, DOI: 10.1038/15271
- CAMPEAN ET AL: "CD40-CD154 expression in calcified and non-calcified coronary lesions of patients with chronic renal failure", ATHEROSCLEROSIS, ELSEVIER, AMSTERDAM, NL, vol. 190, no. 1, 15 December 2006 (2006-12-15), pages 156 - 166, XP005805456, ISSN: 0021-9150, DOI: 10.1016/J.ATHEROSCLEROSIS.2006.01.014
- HARVEY ET AL.: "Interferon-.gamma. and atherosclerosis: Pro- or anti-atherogenic?", CARDIOVASCULAR RESEARCH, vol. 67, no. 1, 2005, pages 11 - 20, XP027645363

## Description

The present disclosure relates to peptides for use in methods for the reduction of LDL cholesterol, wherein the peptide inhibits the interaction of CD40 and CD154.

### Background

According to the World Health Organization (WHO), an estimated 17.7 million people died from cardiovascular diseases (CVDs) in 2015, which represented 31% of all global deaths. CVDs, may also be known as heart and blood vessel disease, and includes numerous problems, many of which are related to a process called atherosclerosis. Moreover, according to the Healthcare Cost and Utilization Project (HCUP) which is sponsored by the Agency for Healthcare Research and Quality (AHRQ), in 2011, coronary atherosclerosis alone accounted for more than $10.4 billion in hospital costs. Accordingly, in 2011, coronary atherosclerosis alone was one of the ten most expensive conditions for inpatient hospitalizations in the United States.

Atherosclerosis is defined by arterial plaque formation that may lead to heart attack and stroke. Arterial plaque formation is caused by the deposition of cells, substances, waste products, and cellular debris including, but not limited to: cholesterol, dead cells, dendritic cells, foam cells, macrophages, mast cells, monocytes, smooth muscle cells, T-cells, collagen, calcium, and fibrin. Inflammatory changes within the arterial wall and plaque may play a crucial and causative role in atherosclerotic disease development. Consequently, the concept of atherosclerosis as an autoimmune and inflammatory disease has been investigated; however, a therapeutic control has not been established. The importance of controlling inflammation is highlighted by current clinical trials targeting other aspects of autoimmune, inflammation, and cardiovascular disease and death.

For example, CIRT (Cardiovascular Inflammation Reduction Trial) is attempting to use methotrexate to target interleukin-6 (IL-6) to test whether methotrexate will reduce rates of myocardial infarction, stroke, and cardiovascular death among patients with coronary artery disease patients with type 2 diabetes. Another example includes, CANTOS (Canakinumab Anti-inflammatory Thrombosis Outcomes Study) which is studying whether canakinumab can block the pro-inflammatory cytokine interleukin - 1β (IL-1β) to reduce rates of recurrent myocardial infarction, stroke, and cardiovascular death rates in heart attack patients who remain at a high risk because of elevated levels of the inflammatory biomarker high sensitivity C-reactive protein (hsCRP). These studies acknowledge that inflammation plays a critical role in atherothrombosis and atherosclerosis; however, these studies also recognize that is unknown whether inhibition of inflammation per se will lower vascular event rates.

Mammalian and human atherosclerotic lesions are characterized as a chronic inflammatory-fibroproliferative disease of the blood vessel wall containing monocytes, macrophages, endothelial cells, smooth muscle cells, platelets, and T-cells. Each of these cell types can express either or both of the CD40/CD154 costimulatory pair. This dyad is responsible for enhancing the immune response and may contribute to many chronic inflammatory diseases including rheumatoid arthritis, multiple sclerosis, and type 1 diabetes (T1D). However, no viable therapy exists for this highly atherogenic dyad.

Inflammation may occur when inflammatory cells, such as neutrophils, eosinophils, basophils, mast cells, macrophages, platelets, and endothelial cells, respond to inflammatory events or harmful stimuli, such as, invading microorganisms, damages cells, or other irritants. The body's inflammatory response is beneficial because for example, in the case of invading microorganisms, the inflammatory response is an important step in localizing the infecting agent for removal by the immune system. However, in autoimmunity there is no infection, yet severe inflammation is present or persistent. The inflammation in this case, referred to as aseptic chronic inflammation (ACI), is detrimental since it destroys normal tissues. The results of this aseptic inflammation are life-altering and in some cases life-threatening. Moreover, as with acute inflammation, this process is mediated by immune cells, including T-cells.

A major concern for modern medicine is how to control ACI such as that which occurs during autoimmune diseases, as well as how to control acute inflammation resulting from trauma. Inflammation, both chronic and acute, leads to tissue degeneration and eventual loss of function of major organs. ACI is not limited to a single disease, but is instrumental in numerous autoimmune diseases, including, but not limited to: type 1 diabetes (T1D), multiple sclerosis (MS), systemic lupus erythematosus (SLE), rheumatoid arthritis (RA), Crohn's disease, inflammatory bowel disease (IBS), chronic obstructive pulmonary disease (COPD) including types of autoimmune asthma, atherosclerosis, vasculitis, hypertension, thyroiditis including Hashimoto's and Graves diseases, primary biliary cirrhosis, Paget's disease, Addison's disease, acute respiratory distress syndrome (ARDS), acute lung injury, and aseptic chronic inflammation (ACI) associated with organ transplantation.

Autoimmune disorders are classified into two types: organ-specific (directed mainly at one organ) and non-organ-specific (widely spread throughout the body). Examples of organ-specific autoimmune disorders are insulin-dependent Type 1 diabetes (T1D) which affects the pancreas; Hashimoto's thyroiditis and Graves' disease, which affect the thyroid gland; pernicious anemia, which affects the blood; Addison's disease, which affects the adrenal glands; chronic active hepatitis, which affects the liver; myasthenia gravis which affects the muscle; and multiple sclerosis (MS), which affects tissue of the nervous system. An example of a non-organ-specific autoimmune disorders is rheumatoid arthritis (RA).Autoimmune diseases are often chronic, debilitating, and life-threatening. The National Institutes of Health (NIH) estimates that up to 23.5 million Americans suffer from autoimmune disease and that the prevalence is rising. It has been estimated that autoimmune diseases are among the ten leading causes of death among women in all age groups up to 65 years.

Acute inflammation, as observed during trauma or sepsis, is also immune cell mediated. While a comprehensive, complete, and exhaustive list of the molecular mediators in this process have not yet been identified, a prominent role for T-cells, lymphocytes, neutrophils, macrophages, monocytes, neutrophils, eosinophils, basophils, mast cells, and other inflammatory cells is strongly implicated. Therefore, a process to modulate these cell types may control the inflammatory response.

A unique T cell subset has been shown to be instrumental in the development of autoimmune disease. These cells are phenotypically characterized as CD4loCD40+ (Waid, D.M., et al., Eur. J. of Immunol., 34:1488, 2004; Vaitaitis, G.M., et al., Cutting Edge, J. Immunol., 170:3455, 2003; Wagner, D.H., Jr., et al., Proc. Nat'l. Acad. Sci. USA, 99:3782, 2002; Wagner, D.H., Jr., et al., Int'l J. of Mol. Med. 4:231, 1999), and are referred to as Th40 cells. (Waid, D.M., et al. (2004) Eur. J. of Immunol. 34:1488; Vaitaitis, G.M., et al., Cutting Edge, J. Immunol. 170:3455, 2003; Wagner, D.H., Jr., et al., Proc. Nat'l Acad. Sci. USA 99:3782, 2002; Wagner, D.H., Jr., et al., Int'l J. of Mol. Med. 4:231, 1999). CD40 expression typically is associated with antigen presenting cells and the majority of prior art describes CD40 as being expressed on B cells, macrophages, monocytes, and other cells; however, CD40 proteins are also expressed on T cells (Waid, D.M., et al., 2004. Eur. J. of Immunol., 34:1488, 2004; Vaitaitis, G.M., et al., Cutting Edge, J. Immunol., 170:3455, 2003; Wagner, D.H., Jr., et al., Proc. Nat'l Acad. Sci. USA, 99:3782, 2002; Wagner, D.H., et al., Int'l. J. of Mol. Med., 4:231, 1999; Bourgeois, C., et al., Science, 297:2060, 2002; Fanslow, W.C., et al., J. of Immun., 152:4262, 1994; Ramsdell, F., et al., J. of Immunol. 152:2190, 1994; Grabstein, K.H., et al., J. of Immunol., 150:3141, 1993; Armitage, RJ., et al., Sem. in Immun., 5:401, 1993; Cooper, C.J., et al., J of Immunol., 173:6532, 2004). While Th40 cells comprise a proportion of the peripheral CD4+ compartment in naive, non-autoimmune mice (Waid, D.M., et al., Eur. J. of Immunol., 34:1488, 2004; Wagner, D.H., Jr., et al., Int'l J. of Mol. Med., 4:231, 1999), and in humans (Waid. D.M., et al., Clin. Immunol. 124:138, 2007), this proportion is drastically expanded to as much as 50% of the CD4+ compartment in autoimmune prone mice (Waid, D.M., et al., Eur. J. of Immunol. 34:1488, 2004; Wagner, D.H., Jr., et al., Proc. Nat'l Acad. Sci. USA 99:3782, 2002; Wagner, D.H., et al., Int'l J. of Mol. Med., 4:231, 1999) and humans (Waid, D.M., et al., Eur. J. of Immunol. 34:1488, 2004; Waid. D.M., et al., Clin. Immunol. 124:138, 2007; Waid. D.M., et al., Clin. Immunol. 124:138, 2007). These T cells do not express early activation markers and occur in the naive phenotype of non-challenged mice.

In NOD (non-obese diabetic) mice, Th40 cells occur at exaggerated levels in spleen, lymph nodes and the pancreas, even prior to diabetes onset (Waid, D.M., et al., Eur. J. of Immunol. 34:1488, 2004; Wagner, D.H., Jr., et al., Proc. Nat'l Acad. Sci. USA 99:3782, 2002). An elevated number and percentage of these T cells are seen in peripheral blood of type 1 diabetic (T1D) patients when compared to non-autoimmune controls and type 2 diabetic patients (Waid. D.M, et al., Clin. Immunol., 124:138, 2007).

The observed increase in Th40 cells could mean that those T cells are antigen responsive or that CD40 expression is activation induced. Furthermore, several diabetogenic T cell clones are CD40+ (Wagner, D.H., Jr., et al., Proc. Nat'l Acad. Sci. USA 99:3782, 2002). Purified primary Th40 cells from NOD mice and from pre-diabetic NOD (12 - weeks of age) mice successfully transfer type 1 diabetes to NOD/scid (Non-Obese Diabetic/Severe Combined Immunodeficiency) recipient mice, directly demonstrating pathogenicity of the Th40 T cell subset (Waid, D.M., et al., Eur. J. of Immunol. 34:1488, 2004; Wagner, D.H., Jr., et al., 2002. Proc. Nat'l Acad. Sci. USA, 99:3782, 2002). It has been shown that Th40 cells infiltrate islet beta cells destroying insulin production thus suggesting islet antigen specificity (Waid, D.M., et al., Eur. J. of Immunol. 34:1488, 2004; Wagner, D.H., Jr., et al., Proc. Nat'l Acad. Sci. USA 99:3782, 2002). It has also been shown that Th40 cells are required for diabetes transfer. Peripheral (spleen and regional lymph node) T cells that were CD40 depleted, then CD25, Treg, depleted were not capable of transferring diabetes to Scid (Severe Combined Immunodeficiency) recipients. Even though Treg cells were removed, if the auto-aggressive CD40+ T cells subset is absent, disease transfer does not occur.

While Th40 cells are important in the development of autoimmunity, another important factor is expression of the CD40 - Ligand, CD154. CD154 is temporally induced on activated T-cells in response to CD3/TCR stimulation (Lederman, S. et al., J. of Exp. Med., 175:1091, 1992). CD154 expression has also been demonstrated on platelets, monocytes, basophils, eosinophils, dendritic cells, fibroblasts, smooth muscle, and endothelial cells (Russo, S. et al., J. Immunol. 171:5489, 2003; Stumpf, C., et al., Eur. J. Heart Fail., 5:629, 2003; Schonbeck, U., et al., Cell Mol. Life Sci. 58:4, 2001). CD154 is a member of the tumor necrosis factor (TNF) super-family and a soluble form of CD154 (sCD154) has been described (Russo, S., et al., J. Immunol. 171:5489 2003; Stumpf, C., et al., Eur. J. Heart Fail 5:629, 2003; Toubi, E., et al., Autoimmunity 37:457, 2004). Therefore, sCD154 may act like a cytokine (Stumpf, C., et al., Eur. J. Heart Fail. 5:629, 2003). Even though CD154 has not been genetically linked in T1D studies, sCD154 is significantly elevated in T1D and may play a role in the disease process (Varo, N. et al., Circulation 107:2664, 2003; Cipollone, F., et al., Diabetologia 48:1216, 2005; Devaraj, S., et al., Diabetes 55:774, 2006). The importance of CD40-CD154 interaction in autoimmunity has been established (Wagner, D.H., Jr., et al., Proc. Nat'l Acad. Sci. USA 99:3782, 2002; Kobata, T., et al., Rev. Immunogenet. 2:74, 2000; Homann, D., et al., Immunity 16:403, 2002; Goodnow, C. C., et al., Lancet 357:2115, 2001; Balasa, B., et al., J. of Immunol. 159:4620, 1997). Blocking CD40-CD154 interaction may prevent collagen induced arthritis, (Durie, F.H., et al., Science 281:1328, 1993) experimental autoimmune encephalitis (Howard, L.M., et al., Autoimmunity 37:411, 2004), prostatitis (Grossman, M.E., et al., J. Immunother. 24:237, 2001), and type-1 diabetes in the NOD mouse model (Durie, F.H. et al., Science 281:1328, 1993; Balasa, B. et al., Journal of Immunology 159:4620, 1997; Howard, L.M., et al., Autoimmunity 37:411, 2004; Grossman, M.E. et al., J. Immunother. 24:237, 2001). In the diabetes model, it was essential to administer a CD154 blocking antibody to NOD mice at 3-weeks of age because at 9-weeks, blocking antibodies had no effect on diabetes prevention (Balasa, B. et al., J. of Immunol. 159:4620, 1997).

Previous work has also demonstrated that the Th40 cell subset induces RAG1 and RAG2 (Recombination-Activating Genes) transcription, translation and nuclear translocation (Vaitaitis, G.M., et al., Cutting Edge, J. Immunol. 170:3455, 2003) when CD40 is engaged (Vaitaitis, G.M. et al., Cutting Edge, J. Immunol. 170:3455, 2003). CD3 engagement does not induce RAG1 or RAG2 in T-cells (Vaitaitis, G.M., et al., Cutting Edge, J. Immunol. 170:3455, 2003). Subsequent to RAG1/RAG2 induction, CD40-mediated T-cell receptor (TCR) revision occurs in peripheral T cells (Vaitaitis, G.M. et al., Cutting Edge, J. Immunol. 170:3455, 2003). CD40 induction of TCR revision is RAG dependent. T cells isolated from a TCR-Tg mouse undergo TCR revision when CD40 engaged, but T-cells from the TCR-Tg.RAG-/- mouse do not TCR revise when CD40 engaged (Wagner, D.H., Jr. et al., Int'l J. of Mol. Med. 4:231, 1999).

CD40 is a 50-kDa integral membrane protein of the tumor necrosis factor receptor (TNF-R) family. It is constitutively expressed as a homotrimer (Foy TM, et al., Ann. Rev. of Immunol., 14:591, 1996). In general, stimulation of all CD40-expressing cell types induces operations which contribute to inflammation, such as enhancement of costimulatory and adhesion molecules, and up-regulation of proteolytic enzymes (Mach, F. et al., Atherosclerosis. 137 Suppl: S89-95, 1998).

CD40's ligand - CD154 - is a 39-kDa protein that belongs to the tumor necrosis factor (TNF) family. CD40 forms a trimer that binds CD154 at the interface of the three monomers. CD154 is expressed commonly on cells beyond the surface-expressed CD154, as CD154 may also exist in a soluble biologically active form (sCD154) that is shed from the cell surface after activation. The main source of sCD154 is platelets. (Foy TM, et al., Ann. Rev. of Immunol., 14:591, 1996).

Genetically manipulated mouse models are utilized for research and development concerning atherosclerosis because wild type mice are generally highly resistant to development and progression of atherosclerosis. Prior studies have attempted to block the CD40/CD154 interaction by using monoclonal antibodies and this approach has proven efficacious in several mouse model studies utilizing the Apoe-/- or LDLr deficient atherosclerotic models. Additionally, these same mouse models built with a deletion of CD154 saw significant reductions in overall plaque formation and may have also contributed to production of a more stable plaque phenotype. Clinically, stable plaques are identifiable and denoted by increased collagen and smooth muscle content, a thick fibrous cap, and an observable decrease in T cell, macrophage, and lipid accumulation.

Multiple treatment options have been put forward to address and control both chronic and acute inflammation. Many approaches use non-steroidal anti-inflammatory drugs (NSAIDS) that attack the production of leukotrienes and prostaglandins, cellular products that cause localized inflammation. Other approaches use more powerful immunosuppressant drugs such as cyclophosphamide, methotrexate and azathioprine that suppress the immune response and stop the progression of the disease. Still other treatments involve the use of monoclonal antibodies (mAb) designed to alter the immune responses to self-tissues, as occurs during autoimmune diseases. However, all of these treatments often have severe, long-term side effects.

Current immune-modulatory therapies may rely upon monoclonal antibody treatments that may give rise to complications. For example, antibodies administered to a subject may cross-react with unintended targets and cause severe nephritic complications and those that specifically act against CD154 may cause embolic complications. Further, the CD40-CD154 interaction may play an important role in antibody generation which may indicate that administration of a monoclonal antibody could induce auto-antibody generation and further complications, which may inhibit the restoration of normal immune function (see generally Banchereau, J. et al., Annu. Rev. of Immunol. 12:881, 1994).

Other studies have demonstrated that blocking the CD154 interaction by using monoclonal antibodies, or limiting the CD40 receptor by monoclonal antibodies may abrogate atherosclerosis, and may confer a more favorable plaque phenotype characterized by lower inflammation and higher fibrosis. These studies additionally demonstrated that neointimal formation and restenosis may be limited by blocking the CD154 interaction. Studies concerning lupus nephritis may have demonstrated that blocking CD40 mediated signals can reduce anti-double-stranded DNA (anti-dsDNA) antibodies. Moreover, these studies may demonstrate that the reduction of anti-dsDNA was associated with increased serum complement levels and reduced glomerular inflammation, which may be viewed positively from a clinical perspective. However, the use of monoclonal antibodies to target the CD40/CD154 dyad was abandoned due to thromboembolic events which may have been related to the functioning of CD154 in thrombus stabilization. It is postulated that CD154 stabilize thrombi by interaction with the integrin α_{IIb}β₃, and by inhibiting CD154, thrombi may be less stable, and as a consequence shed emboli causing thrombotic events.

US 2013/0236495 discloses peptides that bind to CD40 protein so that they affect the interaction of CD40 and CD154 to reduce inflammation in diseases.

There exists a need in the art for compounds that can reduce LDL cholesterol in subjects in the need thereof. The present developments may address this need by describing peptides for use in methods for reducing LDL cholesterol comprising the administration of a therapeutically effective amount of CD40 peptides. Further, the present developments may provide the added benefit of preventing auto-antibody generation, and thus allow the resumption of normal immune function.

This statement of background is for information purposes only and is not intended to be a complete or exhaustive explication of all potentially relevant background.

### Summary

The present developments provide peptides for use in novel methods for reducing LDL cholesterol. Atherosclerosis may arise as a result of chronic inflammatory response of white blood cells in the walls of arteries. It is postulated that the chronic inflammatory response may and the subsequent buildups of plaque in arteries may be caused by elevated levels of cholesterol and triglycerides in the blood, high blood pressure, and cigarette smoking.

The present developments are based on the knowledge that interaction of CD40-ligand (CD154 protein) with CD40 protein expressed on T-cells (Th40 cells), may be important in the development of atherosclerosis and autoimmune disease. The present developments may be based on the elucidation of the critical residues in CD40 and CD154 that may be important for this interaction. The present developments relate to blocking the interaction between a CD40 protein and a CD154 protein through the use of small peptides that interact with the CD40 protein at a site where the CD154 protein would normally bind.

Preferred peptides for use of the invention as defined herein are those that are less than 25 amino acids in length, and that bind to a CD40 protein, thereby inhibiting its interaction with a CD154 protein.

In an embodiment, the peptide for use may bind to a CD40 protein with a Kd of greater than 10⁶. Further, in this embodiment, the peptide for use may affect the interaction between CD40 and CD154. Additionally, a preferred embodiment may inhibit the binding of CD40 to CD154. Moreover, in this embodiment, the peptide for use binds to CD40 at the site where CD40 interacts with CD154.

An aspect of the present developments, is a peptide for use in a method to lower LDL cholesterol in a subject, wherein the peptide specifically binds CD40 presenting cell at the CD154 binding site, wherein the method comprising administering to the subject in need thereof a therapeutically effective amount of the peptide and wherein said peptide comprises an amino acid sequence selected from SEQ ID NO:3, SEQ ID NO:4, SEQ ID NO:5, SEQ ID NO:6, SEQ ID NO:8 and SEQ ID NO:9. The invention is set out in the appended set of claims.

### Brief Description of the Figures

Fig. 1 is a chart of the effect of various peptides of CD154 on the development of diabetes in NOD mice. The 6-mer (SEQ ID NO: 4), 8-mer (SEQ ID NO: 5), 10-mer (SEQ NO: 24), 13-mer (SEQ ID NO:25), 15-mer (SEQ ID NO: 7), and 24-mer (SEQ ID NO:26) were tested.
Fig. 2A is a chart of the effect of a 15-mer peptide from CD154 on the CD4/CD8 ratio in NOD mice.
Fig. 2B is a chart of the effect of 15-mer peptide in treated versus control pancreata excised, examined, and scored.
Fig. 3 is a graph of reversal of diabetes in NOD mice using a 15-mer peptide from CD154.
Fig. 4 is a dot-plot of the detection of Th40 cells using a 15-mer peptide from CD154.
Fig. 5 is a dot-plot of a screening of B cells using a 15-mer peptide from CD154.
Fig. 6 is a chart demonstrating a comparison of Th40 cell levels in diabetic and non-diabetic mice.
Fig. 7 is a chart demonstrating the effect of treatment with the 15-mer peptide on insulin granulation of the pancreas.
Fig. 8 is a graph that shows the effect of mutations in the 15-mer peptide on the ability of the 15-mer peptide to inhibit development of diabetes in NOD mice.
Fig. 9 is a chart showing the number of cells (×10⁶) of CD3+CD4+CD40+ in different mice models.
Fig. 10 is a chart showing the percentage of TH40 cells in the peripheral blood in human subjects in control and diabetic subjects.
Fig. 11 is a dot-plot comparing CD4+ and CD40 cell data obtained through flow cytometry.
Fig. 12 is a chart showing the percentage of Th40 cells of CD3+CD4+ population of mice models.
Fig. 13 is a chart that shows Th40 Cell count in peripheral blood of ApoE-/- mice
Fig. 14 is an image at 200x magnification showing Th40 cells in the shoulder region of plaque in the ApoE-/- mouse model.

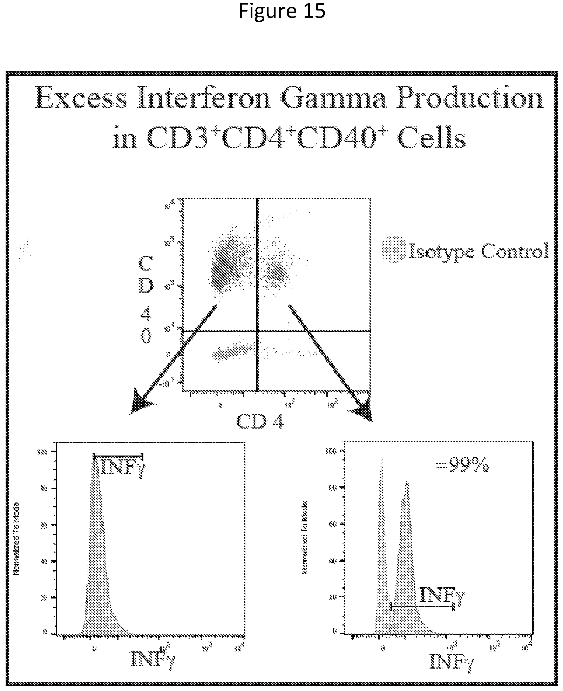

Fig. 15 is a graph of interferon gamma control of Th40 proliferation.
Fig. 16 is a plot of excess interferon gamma production in CD3+CD4+CD40+ cells.
Fig. 17 is a sample of stains of aortic arch of the lesser curvature of the aortic arch.
Fig. 18 is a stain of lesser curvature of aortic arch in control and treated subjects.
Fig. 19 is a chart of area measurements of the lesser curvature of aortic arches and plaques.
Fig. 20-23 are charts of treated and control ApoE mice subjects.
Fig. 23 is a chart of the LDL Cholesterol level in untreated and treated mice.
Fig. 24 is a table of blood clot data in human subjects.

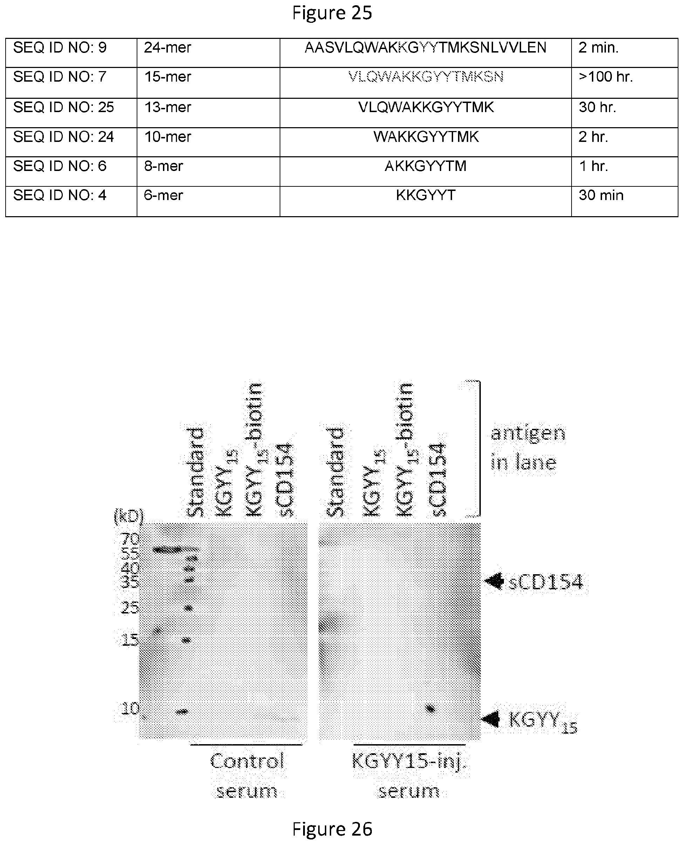

Fig. 25 is a table providing the relative peptide stability assessed by ExPaSY analysis.
Fig. 26 is a western blot comparing control and treated samples from subject mice.
Fig. 27 is a graph of LDL cholesterol measured in treated and untreated subjects.
Fig. 28A is an image of KGYY6 treated aortic en-face Sudan IV staining.
Fig. 28B is an image of control aortic en-face Sudan IV staining.
Fig. 29 is a graph demonstrating the reduction of lesion areas of Sudan IV staining.
Fig. 30 is a graph of plaque volume reduction for area under the curve.
Fig. 31 is a graph of plaque composition for KGYY6 treated and control subject mice.
Fig. 32A is an image of trichrome stained sections of KGYY6 treated subject.
Fig. 32B is an image of trichrome stained sections of control subjects.

### Detailed Description

The present subject matter is based on the discovery that a unique subset of T-cells, which express CD40 protein, and thus are referred to as Th40 cells, that may be instrumental in autoimmune inflammation. Moreover, involvement of Th40 cells in the autoimmune process may be dependent on the interaction between CD40 protein expressed on the surface of the T-cell, and CD154 protein. Interaction of CD40 and CD154 results in activation signals being delivered between the cells, and subsequent activation of the Th40 cell. Such activation results in propagation of the Th40 cell and an increase in inflammation (e.g., an increase in the number of immune cells and immunoregulatory molecules, present in the system). Accordingly, inhibition of the CD40/CD154 interaction can modulate Th40 cell activity, and thereby affect inflammation. Thus, the present subject matter relates to the peptides, and administration thereof, that may affect the interaction between a CD40 protein and a CD154 protein, thereby modulating inflammation.

Before the present development is further described, it is to be understood that this invention is not strictly limited to particular embodiments described, as such may of course vary. It is also to be understood that the terminology used herein is for the purpose of describing particular embodiments only, and is not intended to be limiting, since the scope of the present invention will be limited only by the claims.

It must be noted that as used herein and in the appended claims, the singular forms "a," "an," and "the" include plural referents unless the context clearly dictates otherwise. It should further be understood that as used herein, the term "a" entity or "an" entity refers to one or more of that entity. For example, a nucleic acid molecule refers to one or more nucleic acid molecules. As such, the terms "a", "an", "one or more" and "at least one" can be used interchangeably. Similarly, the terms "comprising", "including" and "having" can be used interchangeably.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments are specifically embraced by the present invention and are disclosed herein just as if each and every combination was individually and explicitly disclosed. In addition, all sub-combinations are also specifically embraced by the present invention and are disclosed herein just as if each and every such sub-combination was individually and explicitly disclosed herein.

It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Furthermore, as used herein the term animal refers to a vertebrate, preferably a mammal, more preferably a human. Suitable mammals on which to use the methods of the present invention include but are not limited farm animals, sports animals, pets, primates, mice, rats, horses, dogs, cats, and humans. The term animal can be used interchangeably with the terms subject or patient.

As used herein, the terms interact, interaction, and the like, mean that two molecules come into sufficient physical proximity such that they cause a modulation of inflammation. One such type of interaction is a binding interaction. In such an interaction the peptide associates with CD40 to form a complex. An example of complex formation is the association of an antigen with an antibody. According to the present subject matter, binding of a peptide hereof to a CD40 protein can be reversible (e.g., non-covalent binding interactions) or non-reversible (e.g., covalent binding interactions). Moreover, a reversible interaction can be strong or weak, the strength of the interaction being determined by the forces (e.g., ionic charges, hydrogen binding, van der Walls interactions, etc.) exerted by each protein on the other protein in the complex. Factors affecting the strength of an interaction between two molecules are known to those skilled in the art. One useful measure of the strength of binding between two molecules, such as a peptide and a protein, is the dissociation constant (Kd). Preferred peptides for use of the present invention are those that bind to a CD40 protein with a Kd of no more than about 1 x 10⁻⁶ M, about 1 x 10⁻⁷ M, or about 1 x 10⁻⁸ M. Particularly preferred peptides for use are those having a Kd of less than about 1 x 10⁻⁹ M. In one embodiment, a peptide for use hereof binds to a CD40 protein with a Kd of less than 100 nM, less than 50 nM, less than 25 nM, less than 10 nM, less than 5 nM, less than 3 nM, less than 2 nM, or less than 1 nM. Methods of measuring and analyzing binding interactions between a peptide and a CD40 protein are known by those of skill in the art.

As used herein, to modulate inflammation means to change the level of Th40 cells present in an animal, or in a culture of T cells. As used herein, the terms level, number, count and concentration can be used interchangeably. Modulation of inflammation can mean an increase or decrease in the number of Th40 cells present in the inflammatory environment. Consequently, modulation can be referred to as positive or negative. Positive modulation (also referred to as up-regulation) of inflammation refers to an increase in the number of Th40 cells in the inflammatory environment. Negative modulation (also referred to as down-regulation) of inflammation refers to a reduction in the number of Th40 cells present in the inflammatory environment. A preferred peptide is one that down-regulates inflammation, thereby reducing the number of Th40 cells present in the inflammatory environment. Positive and negative modulation of inflammation may or may not result in a change in the type and amount of immunoregulatory molecules present in the inflammatory environment.

It will be appreciated by those skilled in the art that both a cell culture system and the immune system of an animal comprise basal levels of immune cells and immunoregulatory molecules. The phrases basal level and normal level can be used interchangeably. With regard to the immune system of an animal, as used herein, the basal level of a type of immune cell (e.g., Th40 cell), or a immunoregulatory molecule, refers to the average number of that cell type, or immunoregulatory molecule, present in a population of individuals considered healthy (i.e., free of metabolic, autoimmune, or infectious disease). With regard to a cell culture system, as used herein, the basal level of a type of immune cell, or an immunoregulatory molecule, refers to the average level of that cell type, or immunoregulatory molecule, present in a population of cells that is non-activated. Those skilled in the art are capable of determining if a T-cell, or a population of such cells, is activated. For example, the expression of CD69, CD25 and/or CD154 proteins by a cell indicates that the cell has been activated.

The basal level of a cell or molecule can be a specific amount (e.g., a specific concentration) or it can encompass a range of amounts. Basal levels, or ranges, of immune cells and immunoregulatory molecules are known to those in the art. For example, in a healthy individual, the normal level of CD4+ T-cells present in human blood is 500-1500 cells/ml. Variability in this measurement can result from differences in the method used to determine the cell count. Furthermore, normal levels of cells can also be reported as a percentage of a total cell population. For example, in a healthy individual, Th40 cells make up less than 25% of the total T cell population. Thus, as used herein, the term inflammation refers to an inflammatory environment in which Th40 cells make up greater than about 25%, greater than about 30%, greater than about 35%, greater than about 40%, greater than about 45% , greater than about 50%, greater than about 55%, greater than about 60%, greater than about 65%, greater than about 70%, greater than about 75%, or greater than about 80% of the total T-cell population. Methods of measuring different types of T-cells in the T-cell population are known to those skilled in the art. Furthermore, a novel method for detecting Th40 cells using peptides hereof is disclosed herein.

As used herein, the phrase inflammatory environment refers to the overall population of immune cells, and related immunoregulatory molecules, that are present in a culture of cells, or in the body of an animal. As such, the phrase inflammatory environment encompasses the types, and/or the relative amounts of immune cells and immunoregulatory molecules (e.g., cytokines) present in a culture of cells, or in an animal, which are involved in affecting an inflammatory reaction. Examples of cells encompassed by the term inflammatory environment include, but are not limited to, T cells, neutrophils, macrophages, granulocytes, and the like. The inflammatory environment relates to cells and molecules that mediate both acute and chronic inflammation. It will be appreciated by those skilled in the art that the inflammatory environment refers to the system to which peptides hereof are administered. In one embodiment, the system is a cell culture system. In one embodiment, the system is a whole animal.

A preferred peptide for use hereof is one that selectively interacts with a CD40 protein in solution, as determined using an assay such as an immunosorbent assay, or on the surface of a T-cell. As used herein, the terms selectively, selective, specific, and the like, indicate the peptide has a greater affinity for a CD40 protein than it does for proteins unrelated to the CD40 protein. More specifically, the terms selectively, selective, specific, and the like indicate that the affinity of the peptide for CD40 is statistically significantly higher than its affinity for a negative control (e.g., an unrelated protein such as albumin) as measured using a standard assay (e.g., ELISA). Suitable techniques for assaying the ability of a peptide to selectively interact with a CD40 protein are known to those skilled in the art. Such assays can be in vitro or in vivo assays. Examples of useful assays include, but are not limited to, an enzyme-linked immunoassay, a competitive enzyme-linked immunoassay, a radioimmunoassay, a fluorescence immunoassay, a chemiluminescent assay, a lateral flow assay, a flow-through assay, an agglutination assay, a particulate-based assay (e.g., using particulates such as, but not limited to, magnetic particles or plastic polymers, such as latex or polystyrene beads), an immunoprecipitation assay, an immunoblot assay (e.g., a western blot), a phosphorescence assay, a flow-through assay, a chromatography assay, a polyacrylamide gel electrophoresis (PAGE)-based assay, a surface plasmon resonance assay, a spectrophotometric assay, a particulate-based assay, an electronic sensory assay and a flow cytometric assay. Methods of performing such assays are well known to those skilled in the art. In one embodiment, an assay can be performed using cells in culture, or it can be performed in a whole animal. Assays can be designed to give qualitative, quantitative or semi-quantitative results, depending on how they are used and the type of result that is desired.

. In an embodiment, the peptide can interact with the CD40 protein such that the strength of the interaction between the CD40 protein and a CD154 protein is decreased. Methods of measuring the strength of binding between the peptide and a CD40 protein are known to those skilled in the art. A preferred peptide for use hereof is one that reduces the strength of the interaction between a CD40 protein and a CD154 protein. Preferred peptides for use hereof reduce the strength of binding between a CD40 protein and a CD154 protein by at least 10%, at least 20%, at least 30%, at least 40%, at least 50%, at least 60%, at least 70%, at least 80%, at least 90%, or at least 95%. A particularly preferred peptide for use is one that completely inhibits binding of CD40 to CD154. Complete inhibition of binding between CD40 and CD154 means that when a peptide hereof is brought into proximity with a CD40 protein and a CD154 protein under conditions that would normally allow the interaction of CD40 and CD154, no such interaction occurs and activation signals are not stimulated in the CD40-expressing cell. Consequently CD40/CD154 mediated modulation of inflammation does not occur. The peptide for use hereof interacts with the CD40 protein at the CD154 binding site. An example of such a peptide is a CD40 ligand competitive antagonist. As used herein, peptides that interfere with, or inhibit, the binding of a CD154 protein to a CD40 protein are referred to as small interfering peptides (SIPs). As used herein a small interfering peptide is a peptide that, through physio-chemical properties, interferes with the interaction of a CD40 protein with a CD154 protein, thereby preventing activation signals from being delivered to the CD40-bearing cell, thus limiting the activation of the CD40-bearing cell, and consequently, inflammation. As demonstrated herein, consequences of such interference are prevention of T-cell activation and propagation, and a prevention or reduction of inflammation. As demonstrated herein, in some instances results of such inhibition or prevention of interaction between CD40 and CD154 may include observable data that demonstrates that atherosclerosis, and characteristics of diseases associated therewith, are prevented, modulated, and/or reduced.

Additionally, a small interfering peptide, may, through its physio-chemical properties, interfere with the interaction of a CD40 protein with a CD154 protein, thereby preventing activation signals from being delivered to the CD40-bearing cell, thus limiting the activation of the CD40-bearing cell, and consequently, modulating, inhibiting, and preventing atherosclerosis. As demonstrated herein, consequences of such interference are prevention of T cell activation and propagation, and a prevention, reduction, or modulation of atherosclerotic developments.

It is understood by those skilled in the art that preferred peptides for use are relatively short since they are easier and less expensive to produce. Preferred peptides for use are those that are less than 20 amino acids in length. A preferred peptide for use is one that is 4, 6, 8, 10, 13, 15, or 24 amino acids in length. In one embodiment, the peptide for use is an amino acid selected from the group of SEQ ID NO:3 (Core-sequence see Table 1), SEQ ID NO:4 (6-mer see Table 1), SEQ ID NO:5 (8-mer mouse see Table 1), SEQ ID NO:6 (8-mer human see Table 1) , SEQ ID NO:8 (15-mer human see Table 1), SEQ ID NO:9 (24-mer see Table 1). The sequences of such peptides are shown below in Table 1.

**Table 1.**

| SEQ ID NO | SEQUENCE | Description |
|---|---|---|
| 1 | MIETYSQPSP RSVATGLPAS MKIFMYLLTV FLITQMIGSV LFAVYLHRRL DKVEEEVNLH EDFVFIKKLK RCNKGEGSLS LLNCEEMRRQ FEDLVKDITL NKEEKKENSF EMQRGDEDPQ IAAHVVSEAN SNAASVLQWA KKGYYTMKSN LVMLENGKQL TVKREGLYYV YTQVTFCSNR EPSSQRPFIV GLWLKPSSGS ERILLKAANT HSSSQLCEQQ SVHLGGVFEL QAGASVFVNV TEASQVIHRV GFSSFGLLKL | SwissPro 27548.2 Mouse CD40 Ligand (CD154 Protein) |
| 2 | | SwissPro 29965 Human CD40 Ligand (CD154 Protein) |
| 3 | KGYY | Core-sequence |
| 4 | KKGYYT | 6-mer |
| 5 | AKKGYYTM | 8-mer-mouse |
| 6 | AEKGYYTM | 8-mer human |
| 7 | VLQWAKKGYYTMKSN | 15-mer-mouse |
| 8 | VLQWAEKGYYTMSNN | 15-mer human |
| 9 | NAASVLQWAKKGYYTMKSNLVMLE | 24-mer |
| 10 | ISQAVHAAHAEINEAGR | 15-mer from ovalbumin; control peptide |
| 11 | G-L-Q-W-A-K-K-G-Y-Y-T-M-K-S-N | Gly-1 |
| 12 | V-G-Q-W-A-K-K-G-Y-Y-T-M-K-S-N | Gly-2 |
| 13 | V-L-G-W-A-K-K-G-Y-Y-T-M-K-S-N | Gly-3 |
| 14 | V-L-Q-G-A-K-K-G-Y-Y-T-M-K-S-N | Gly-4 |
| 15 | V-L-Q-W-G-K-K-G-Y-Y-T-M-K-S-N | Gly-5 |
| 16 | V-L-Q-W-A-G-K-G-Y-Y-T-M-K-S-N | Gly-6 |
| 17 | V-L-Q-W-A-K-G-G-Y-Y-T-M-K-S-N | Gly-7 |
| 18 | V-L-Q-W-A-K-K-G-G-Y-T-M-K-S-N | Gly-8 |
| 19 | V-L-Q-W-A-K-K-G-Y-G-T-M-K-S-N | Gly-9 |
| 20 | V-L-Q-W-A-K-K-G-Y-Y-G-M-K-S-N | Gly-10 |
| 21 | V-L-Q-W-A-K-K-G-Y-Y-T-G-K-S-N | Gly-11 |
| 22 | ISQAVHAAHAEINEAGR | 15-mer from ovalbumin; control peptide |
| 23 | YVQGKANLKSKLMYT | Scrambled peptide |

Interaction of a CD40 protein and a CD154 protein has been shown to occur at particular regions within each protein. The inventors have now shown that, surprisingly, a peptide comprising only a short portion of the CD154 region that interacts with CD40 is capable of binding to a CD40 protein, thereby modulating atherosclerosis.

In one embodiment, the peptide for use is as short as possible yet comprises enough of the CD154 protein to allow interaction with a CD40 protein in such a manner as to modulate atherosclerosis. In one embodiment, a peptide for use hereof comprises 6, 13 or 15 contiguous amino acids from SEQ ID NO:1 or SEQ ID NO:2, and interacts with CD40 in such a manner as to modulate atherosclerosis. The peptide for use comprises a core sequence of lysine-glycine-tyrosine-tyrosine (KGYY; SEQ ID NO:3), which corresponds to amino acids 142-145 of SEQ ID NO:1 and amino acids 143-146 of SEQ ID NO:2. . In one embodiment hereof, a peptide for use comprises at least one sequence selected from SEQ ID NO:3, SEQ ID NO:4, SEQ ID NO:8, and SEQID NO:9, so long as the peptide interacts with CD40 protein in such a manner as to modulate atherosclerosis. In one embodiment of the present subject matter, a peptide for use hereof is a sequence selected from SEQ ID NO:4, SEQ ID NO:8 and SEQID NO:9.

While peptides of the present subject matter may be selected entirely of or from sequences that are responsible for the interaction of the peptide with a CD40 protein, they may additionally contain amino acid sequences that do not interact with a CD40 protein, but which have other useful functions. Any useful, additional amino acid sequence can be added to the CD40-interacting sequence, so long as the additional sequences do not have an unwanted affect on the ability of the CD40 interacting sequence to interact with a CD40 protein. For example, in addition to the amino acid sequence responsible for interacting with a CD40 protein, a peptide hereof can contain amino acid sequences that are useful for visualizing or purifying the peptide. Such sequences act as labels (e.g., enzymes) or tags (antibody binding sites). Examples of such labels and tags include, but are not limited to, B-galactosidase, luciferase, glutathione-s-transferase, thioredoxin, HIS-tags, biotin tags, and fluorescent tags. Other useful sequences for labeling and tagging proteins are known to those of skill in the art.

Likewise, peptides hereof can be modified, so long as such modification does not significantly affect the ability of the peptide to modulate atherosclerosis. Such modifications can be made, for example, to increase the stability, solubility or absorbability of the protein. Examples of such modifications include, but are not limited to pegylation, glycosylation and chemical modification of the peptide.

Peptides hereof may be obtained from nature (e.g., obtained from plants, animals or microorganisms) or they may be produced in a laboratory (e.g., recombinantly or synthetically). Preferred peptides for use are those that are synthesized. Also encompassed are peptides that are combinations of natural and synthetic molecules. General methods for producing and isolating recombinant or synthetic peptides are known to those skilled in the art. It should be noted that, as used herein, an isolated, or biologically pure, molecule, is one that has been removed from its natural milieu. As such the terms isolated, biologically pure, and the like, do not necessarily reflect the extent to which the protein has been purified.

As has been described herein, interaction of the CD40 protein and the CD154 protein are necessary for involvement of Th40 cells in atherosclerosis. Consequently, inhibition of the interaction between a CD40 and CD154 protein using peptides hereof is a useful method of affecting atherosclerosis. In one embodiment hereof, the peptide for use reduces the interaction between the CD40 protein and the CD154 protein by at least 5%, at least 10%, at least 20%, at least 30%, at least 40%, at least 50%, at least 60%, at least 70%, at least 80%, at least 90%, or at least 95%. In one embodiment, the peptide for use reduces the interaction between the CD40 protein and the CD154 protein by a factor of at least 10, at least 100, at least 1,000, at least 10,000. Methods of measuring the strength of the interaction between the CD40 protein and the CD154 protein have been discussed previously, and are also know to those of skill in the art.

Peptides and methods hereof are suitable for use in cell culture as well as for treating a patient. As used herein the term patient refers to any animal in need of such treatment. The animal can be a human or a non-human animal. A preferred animal to treat is a mammal. A peptide can be administered or applied per se, or as pharmaceutical compositions. A peptide for use hereof, or a pharmaceutical composition for use thereof, can be administered to a patient by a variety of routes, including, but limited to, by injection (e.g., intravenous, intramuscular, subcutaneous, intrathecal, intraperitoneal), by inhalation, by oral (e.g., in a pill, tablet, capsule, powder, syrup, solution, suspension, thin film, dispersion or emulsion.), transdermal, transmucosal, pulmonary, buccal, intranasal, sublingual, intracerebral, intravaginal, rectal or topical administration or by any other convenient method known to those of skill in the art.

The amount of a peptide hereof and/or a pharmaceutical composition thereof that will be effective can be determined by standard clinical techniques known in the art. Such an amount is dependent on, among other factors, the patient being treated, including, but not limited to the weight, age, and condition of the patient, the intended effect of the compound, the manner of administration and the judgment of the prescribing physician. Also, in this context, it should be noted that in treating a patient exhibiting a disorder of interest, a therapeutically effective amount of an agent or agents such as these is administered. A therapeutically effective dose refers to that amount of the compound that results in amelioration of one or more symptoms or a prolongation of survival in a patient.

A peptide for use hereof, or a pharmaceutical composition for use thereof, can be administered alone or in combination with one or more other pharmaceutical agents, including other compounds of the present disclosure. The specific pharmaceutical composition depends on the desired mode of administration, as is well known to the skilled artisan.

Because the inventors have discovered that Th40 cells are intimately involved in the development of autoimmune diseases and atherosclerosis, the peptides for use disclosed herein can be used to affect atherosclerosis resulting from such diseases.

One example of a disease that is particularly amenable to treatment using a peptide of the present developments may be atherosclerosis. In atherosclerosis, inflammatory changes of the arterial wall occur resulting in the formation and buildup of arterial plaque. Consequently, control of inflammatory cells and cell signaling via CD40-CD154 interaction may be able to be used to control, modulate, and/or reduce atherosclerotic lesions that are characterized as chronic inflammatory-fibroproliferative disease of the vessel wall. Several murine models of atherosclerosis have been developed. The progression of lesion formation is observable in Apolipoprotein E (ApoE) deficient transgenic mice and can be observed by measurement of the aortic arch, the number and type of plaque, and characterized in accordance with the American Heart Association's staging of atherosclerosis, ranging from AHA type I to AHA type V. AHA type 1, may be characterized by early or initial lesions, may be comprised of histologically "normal" cells, macrophage infiltration, and isolated foam cells. AHA type V, may be characterized by advanced or complicated legions, including but not limited to increased endothelial dysfunction characterized by surface defects, hematoma, hemorrhage, and/or thrombosis.
Moreover, atherosclerosis may be particularly amenable to treatment using a peptide of the current development. The risk for atherosclerosis may result from familial factors (e.g., inheritance) or from other factors, such as the physical condition of the individual. The level of atherosclerotic activity and disease may vary from individual to individual depending on numerous factors such as level of activity, diet, smoking status, and other variable factors that are dynamic such as levels of inflammation. Some methods of risk assessment for atherosclerosis are known to those skilled in the art.
The inventors have also shown that, surprisingly, peptides hereof can be used to reverse the disease process in individuals already showing signs of atherosclerosis. As used herein the phrase to reverse atherosclerosis means to reduce the aortic-arch infiltration, plaque, and lesions of an individual with atherosclerosis to a level comparable to that observably lower levels or in some instances to levels that may be more common in a non-atherosclerotic individual.

As has been described, peptides for use of the present invention selectively bind to a CD40 expressing cell. Consequently, peptides of the present subject matter can be used to identify Th40 cells. **In** general, an assay for detecting Th40 cells using a peptide hereof comprises (a) obtaining a sample of cells; (b) contacting a peptide hereof with said cells under condition suitable to allow binding of the peptide to Th40 cells, if present; (c) washing said cells using conditions that disrupt non-specific interactions, and that remove unbound peptide; and (d) detecting peptide bound to cells. Detection of bound peptide can be achieved directly or indirectly. For example, direct detection can be achieved using a peptide labeled using a detectable marker, as disclosed herein. Following the wash step listed above, the cells are then simply screened for the presence of detectable marker. The presence of detectable marker in the cell sample indicates the presence of Th40 cells, and thus Th40-dependent atherosclerosis. Alternatively, indirect detection involves the use of a second molecule, such as an antibody, that binds to the peptide. **In** an indirect detection assay, following the wash step listed above, a detection molecule that binds the peptide is added to the cell sample. The detection molecule is labeled with a detectable marker. After washing away unbound detection molecule, the cells are screened for the presence of detectable marker. The presence of detectable marker in the cell sample indicates the presence of Th40 cells. It should be understood that the assays described herein are meant as examples of useful assays, and other assay techniques can be employed. Suitable assay techniques are known to those skilled in the art, and are also disclosed in, for example, Molecular Cloning: A Laboratory Manual, Sambrook, J., Fritsch, E.F., and Maniatis, T, Cold Spring Harbor Laboratory Press; 2nd Edition (December 1989).

The assay technology described above can also be used to identify other molecules that affect the interaction of a CD40 protein with a CD514 protein. Examples of such molecules include, but are not limited to, proteins, peptides and small molecules. For example, assays can be designed that test the ability of molecules to compete with a peptide of the present developments for binding to a Th40 cell. For instance, a peptide labeled with a detectable marker, can be mixed with a test molecule and a population of cells known to contain Th40 cells, under conditions that allow binding of the peptide to the Th40 cells. Following an appropriate incubation period, the cells are washed to remove unbound peptide, and the cells screened for the presence of detectable marker. Alternatively, the labeled peptide could be bound to Th40 cells first, and after a wash step to remove unbound peptide, the test molecule could be added to the cells containing bound peptide. Following an incubating period and a wash step to remove unbound molecule, or released peptide, the cells are screened for the presence of detectable marker. In either case, absence of the detectable marker in the cell sample indicates the test molecule is able to compete with the peptide for binding to the Th40 cells, while presence of the detectable marker would indicate the test molecule does not inhibit binding of the peptide to Th40 cells. Inhibition of binding need not be 100%, as such assay would also be useful for identifying molecules that partially inhibit binding of the peptide to Th40 cells. It is understood by those skilled in the art that such assays would involve the use of positive controls (e.g., unlabeled peptide) and negative controls (e.g., a protein/molecule that is known not to bind to Th40 cells).

Because increased levels of Th40 cells are associated with the development of autoimmune disease, the present developments can be used to identify patients at risk for developing autoimmune disease and autoimmune related atherosclerosis and/or cardiovascular disease more generally.

The following examples are provided for the purpose of illustration and are not intended to limit the scope of the present invention.

### Examples

### Example 1 (not part of the invention)

This Example demonstrates the effect of various peptide fragments of CD154 on CD4/CD8 ratios and the development of diabetes in NOD mice.

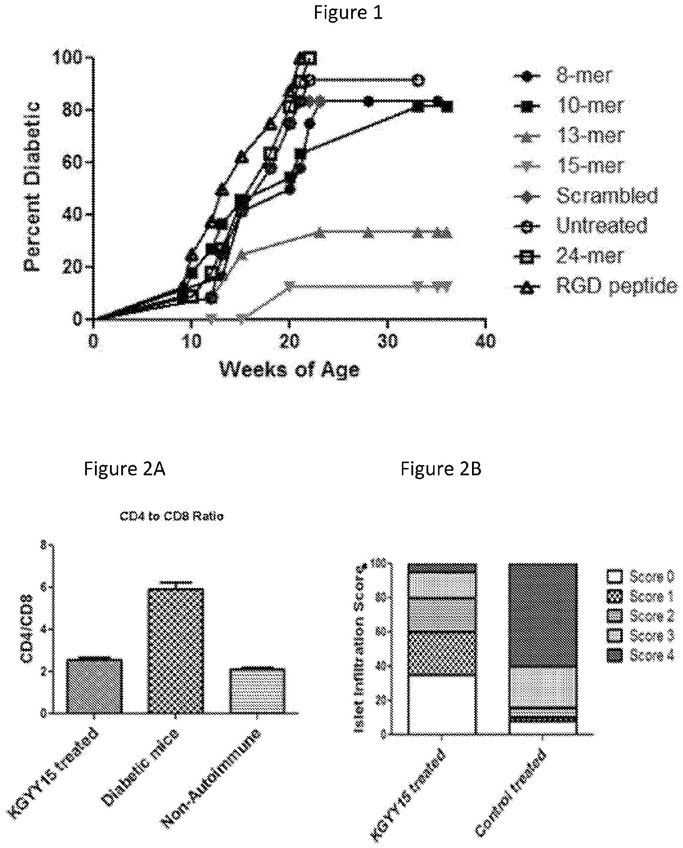

Peptides were designed based on the amino acid sequence of mouse CD154 protein (SEQ ID NO:1) in the SwissPro database. The peptides (8-mer (SEQ ID NO: 5; SEQ ID NO: 6), 10-mer (SEQ ID NO:24), 13-mer (SEQ ID NO:25), 15-mer (SEQ ID NO: 7), 24-mer (SEQ ID NO:26), scrambled (SEQ ID NO: 23), and RGD (arginylglycylaspartic acid) were then ordered from New England Peptide. The RGD peptide is a 15-amino acid sequence from the CD154 sequence that does not include the CD40 binding motif. The lyophilized peptides were suspended in sterile saline at 1 mg/ml. 25 ug (1mg/kg) of a particular peptide was then injected into the tail vein of 6-week old NOD mice. Control mice received 100 ul of sterile saline. This is well before the onset of diabetes (and atherosclerosis), but after damage to pancreatic islets has begun. Weekly after the initial injection, another 100 ug of peptide (or 100 ul of saline in the case of the Control mice) was injected into the tail vein. At 10 weeks of age, mice were monitored for diabetes, as indicated by a blood glucose level greater than 250 mg/dL for three consecutive days. The results of this study are shown in Figure 1. During this time, blood was also taken from the tail vein, or by sub-mandibular venal puncture, and the level of CD4+ and CD8+ cells determined by flow cytometry using antibodies for CD4 protein and CD8 protein. The results of this analysis are shown in Figure 2A.

Pancreata were excised and examined by histology for cellular infiltrates and assigned scores based on observable, measurable, and quantifiable data: 0 = no infiltrate; 1 = one pole infiltrate; 2 = peri-insulitis, bi-polar-infiltrates; 3 = 75% infiltrate and 4 = full infiltration. The results of this analysis are shown in Figure 2B.

The results demonstrate that treatment with a peptide unrelated to the CD154 protein did not reduce the development of diabetes in NOD mice. In contrast, treatment of mice with a 15-mer peptide derived from the CD154 protein prevented the onset of diabetes. Further, the 13-mer peptides derived from the CD154 protein had significant effects on the development of diabetes. In addition, the data demonstrates that the 15-mer peptide did not result in compromise of the immune system, as determined by the CD4/CD8 ratio.

### Example 2 (not part of the invention)

This Example demonstrates the effect of the 15-mer peptide on hyperglycemia in newly diabetic NOD mice.

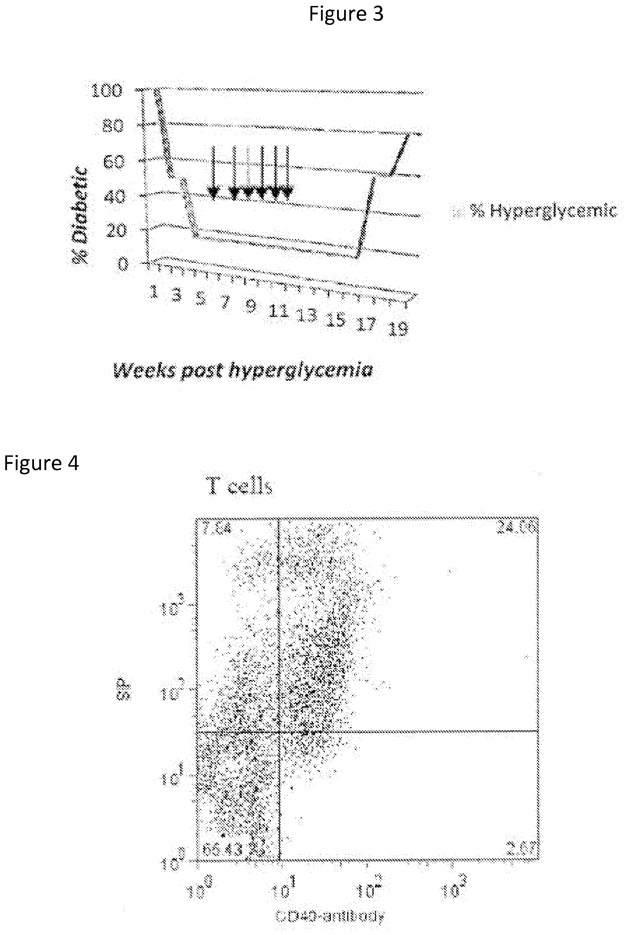

Six mice from that had received the 6-mer peptide in Example 1, and that had subsequently developed diabetes, were injected intravenously with 100 ug of the 15-mer peptide. These mice were then given weekly injections of the 15-mer peptide into their tail veins, and their blood glucose levels monitored twice-weekly. The 15-mer peptide was administered for a total of ten weeks, after which the treatment was stopped. The results of this study are shown in Figure 3.

This study demonstrates that injection of the 15-mer peptide into already diabetic mice can reverse hyperglycemia. It also demonstrates that cessation of the treatment results in return of hyperglycemia within 7 weeks.

### Example 3

This study demonstrates the ability of the 15-mer peptide to bind to Th40 cell and B cells.

Total lymphocytes were isolated from 9 week old NOD mice. The lymphocytes were incubated with anti-CD, anti-CD8, and an FITC-labeled 15-mer peptide, and then analyzed by flow cytometry. Cells were gated for CD4 (both CD4hi and CD4lo populations were included) and CD4 versus the 15-mer peptide. The results of this analysis are shown in Figure 4.

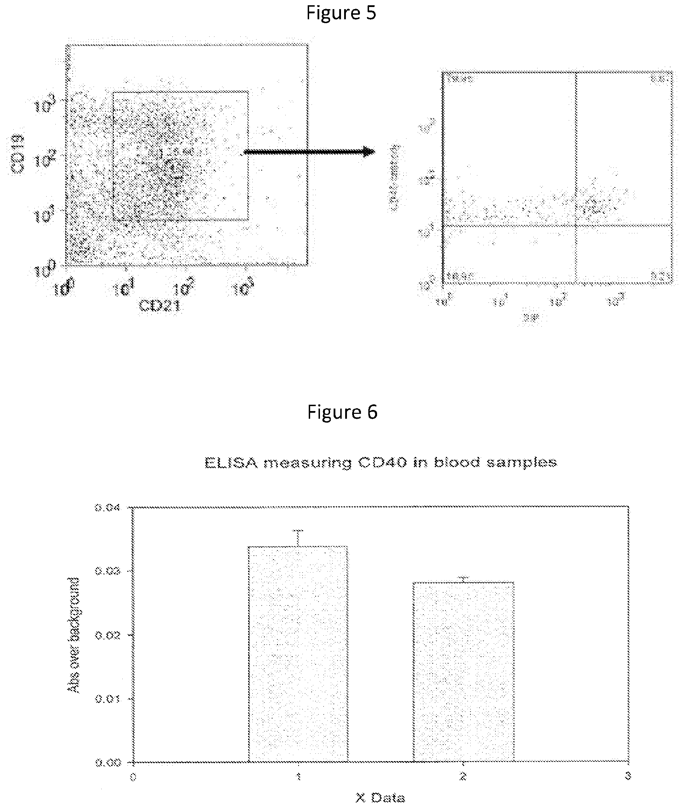

B cells were isolated from the spleens of NOD mice. Sorted MHC-II+ cells were purified from total lymphocytes. Cells were stained with FITC-labeled 15 mer peptide, anti-CD40, and B cell markers CD19 and CD21. MHC-II+ cells were gated for CD19+ and CD21+ and then 15-mer peptide versus CD40 antibody was measured. The results of this study are shown in Figure 5.

This study shows that a substantial majority, 90% of CD40+ T-cells, also bind the 15-mer peptide, thereby demonstrating that the 15-mer peptide is highly specific for CD40+ cells. It also shows that while 90% of B cells were CD40 positive, only 8% of B cells bound the 15-mer peptide.

### Example 4 (not part of the invention)

This example demonstrates the level of CD40 positive cells in the blood of type-I diabetic subjects and non-diabetic (control) subjects.
1 ml of whole blood was obtained from each individual and incubated with biotin-conjugated, 15-mer peptide. The cells were then exposed to horseradish peroxidase (HRP)-avidin, washed and the absorbance at 405 nm determined using a spectrophotometer. The results of this study are shown in Figure 6.

This study demonstrates that blood cells from patients having type-I diabetes had higher 15-mer peptide binding activity than cells from non-diabetic controls.

### Example 5 (not part of the invention)

This example demonstrates the level of insulin granulation observed in the pancreas of NOD mice treated with either the 15-mer peptide or a peptide from ovalbumin.

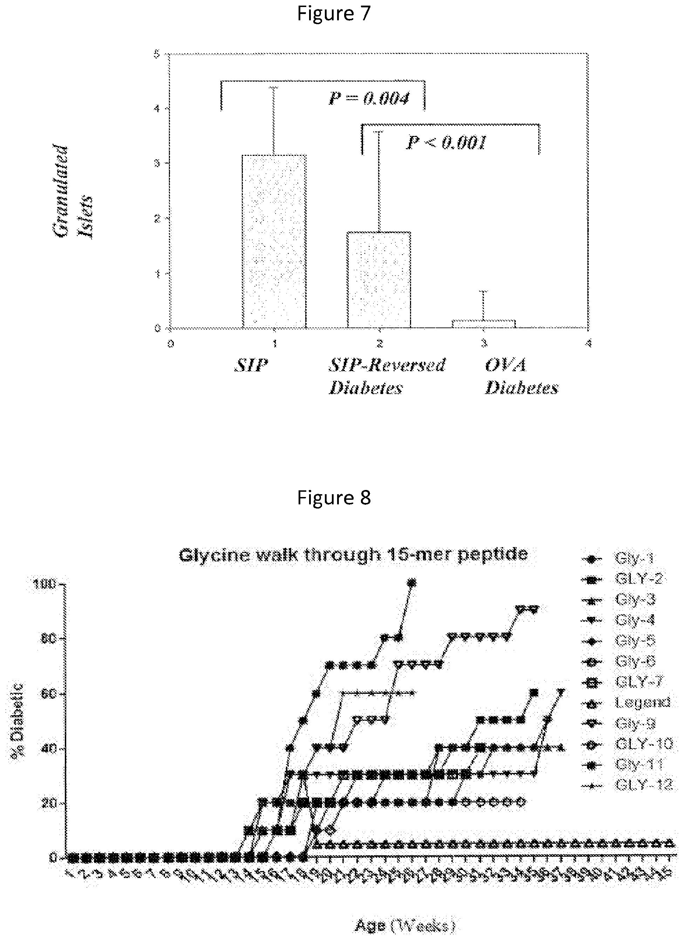

At the onset of diabetes, six NOD mice were injected with 100 ug/ml of the 15-mer peptide (SEQ ID NO:7), resulting in the reversal of hyperglycemia in 80% of the recipients. Six weeks after reversal of hyperglycemia, mice were sacrificed and the pancreas removed for analysis. The pancreas was fixed, sectioned and then stained using an aldehyde/fuschsin stain that allows detection of insulin granules. Granulation of the tissue was scored as follows: 4=completely granulated; 3=75% of islet granulated; 2= 50% of islet granulated, and peri-insulitis; 1=25% of islet granulated; 0= no insulin granules detected. The results of this analysis are shown in Figure 7.

This analysis demonstrates that the 15-mer peptide preserved insulin granules in the majority of the mice, and was significantly improved in peptide-reversed diabetic mice compared to diabetic mice that received an irrelevant peptide.

### Example 6 (not part of the invention)

This example demonstrates the effect of mutations in the 15-mer peptide on its ability to prevent the onset of diabetes.

Peptide were designed and produced as described in Example 1. Variant peptides were produced so that in each variant, a glycine was substituted for an amino acid corresponding to an amino acid in positions 1-9 of SEQ ID NO:7, as follows:
Gly-1 G-L-Q-W-A-K-K-G-Y-Y-T-M-K-S-N (SEQ ID NO:11)
Gly-2 V-G-Q-W-A-K-K-G-Y-Y-T-M-K-S-N (SEQ ID NO:12)
Gly-3 V-L-G-W-A-K-K-G-Y-Y-T-M-K-S-N (SEQ ID NO:13)
Gly-4 V-L-Q-G-A-K-K-G-Y-Y-T-M-K-S-N (SEQ ID NO:14)
Gly-5 V-L-Q-W-G-K-K-G-Y-Y-T-M-K-S-N (SEQ ID NO:15)
Gly-6 V-L-Q-W-A-G-K-G-Y-Y-T-M-K-S-N (SEQ ID NO:16)
Gly-7 V-L-Q-W-A-K-G-G-Y-Y-T-M-K-S-N (SEQ ID NO:17)
Gly-9 V-L-Q-W-A-K-K-G-G-Y-T-M-K-S-N (SEQ ID NO:18)
Gly-10 V-L-Q-W-A-K-K-G-Y-G-T-M-K-S-N (SEQ ID NO:19)
Gly-11 V-L-Q-W-A-K-K-G-Y-Y-G-M-K-S-N (SEQ ID NO:20)
Gly-12 V-L-Q-W-A-K-K-G-Y-Y-T-G-K-S-N (SEQ ID NO:21)

NOD mice were placed in groups of 10, and the mice in each group injected IV weekly with 50 ug of either wild-type (WT; Legend) peptide or a variant peptide (in PBD, pH 7.2) listed above. The development of diabetes was monitored by measuring blood glucose levels on a weekly basis. Mice were considered "diabetic" when blood glucose was 250 mg/dl or greater for 2 consecutive readings. Injections began at 6 weeks of age = pre-diabetes.

This example demonstrates that substitution of a glycine at any of positions 1-7, or 9-12, reduces the ability of the 15-mer peptide to inhibit the development of diabetes. It also shows that such mutations do not completely abolish the ability of the mutated 15-mer peptide to inhibit the development of diabetes.

### Example 7 (not part of the invention)

This example demonstrates that the same elevation of Th40 cell levels in the ApoE deficient mouse model of atherosclerosis is also notably elevated in human Type 1 Diabetes (T1D).

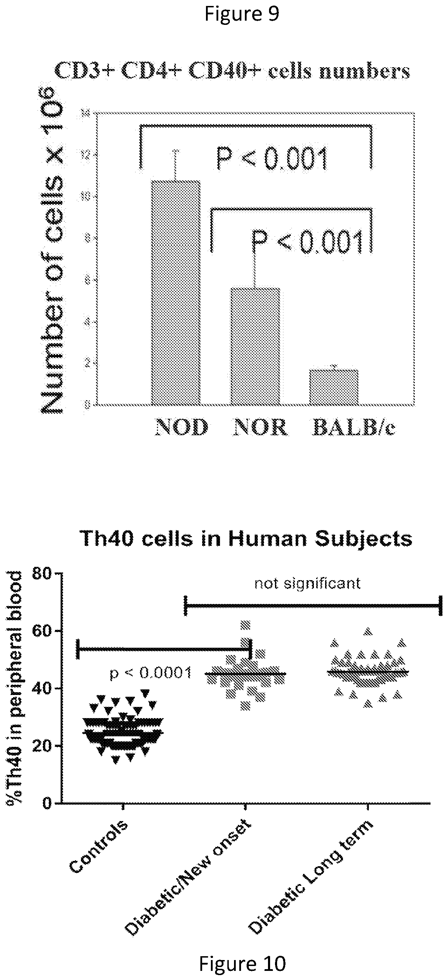

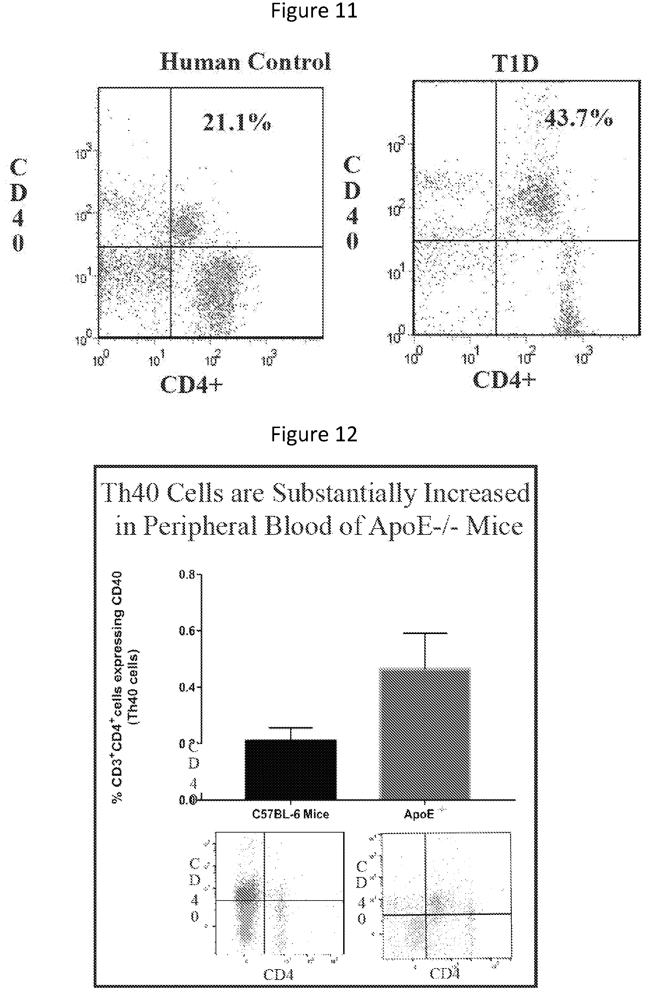

The peripheral blood was measured was measured for total count of CD3+CD4+CD40+ cell numbers in NOD, NOR (non-obese diabetic resistant), and BALB/c (control) mice as in Figure 9. This was compared to the percentage of Th40 cells in peripheral blood in human subjects for control, diabetic/new onset, and long term diabetic populations as in Figure 10. Further, lymphocytes were isolated from 9-week old NOD mice. The lymphocytes were incubated with anti-CD, anti-CD8, and an FITC-labeled 15-mer peptide, and then analyzed by flow cytometry. Cells were gated for CD4 (both CD4hi and CD4lo populations were included) and CD4 versus the 15-mer peptide. These results are displayed in Figure 11.

ApoE deficient mice on a normal chow diet were selected to receive a dose of 1mg/kg of the 15-mer peptide (SEQ ID NO: 7) by IV tail injection, three times a week over a period of 26 weeks, beginning at 9 weeks of age and also utilized a control. At 25 weeks, the animals were euthanized, weighed, and then had blood, spleen, and pancreas removed for analysis. The subjects were then perfused through cardiac puncture with 4% paraformaldehyde. Aortic arches were dissected, dehydrated in sucrose gradient and then flash frozen. Approximately thirty-five 8um longitudinal sections were obtained per mouse for various staining procedures. Flow cytometry was performed utilizing a MACSQuant^{®} Analyzer 10 (Miltenyi Biotec Inc.). Additional analysis was performed using FlowJo ^{®} (FlowJo, LLC wholly owned by BectonDickinson, Inc.) single-cell flow cytometry software.

NOD mice demonstrated increased levels of Th40 cells relative to all CD3+CD4+ cells prior to hyperglycemia as demonstrated in Figure 12.

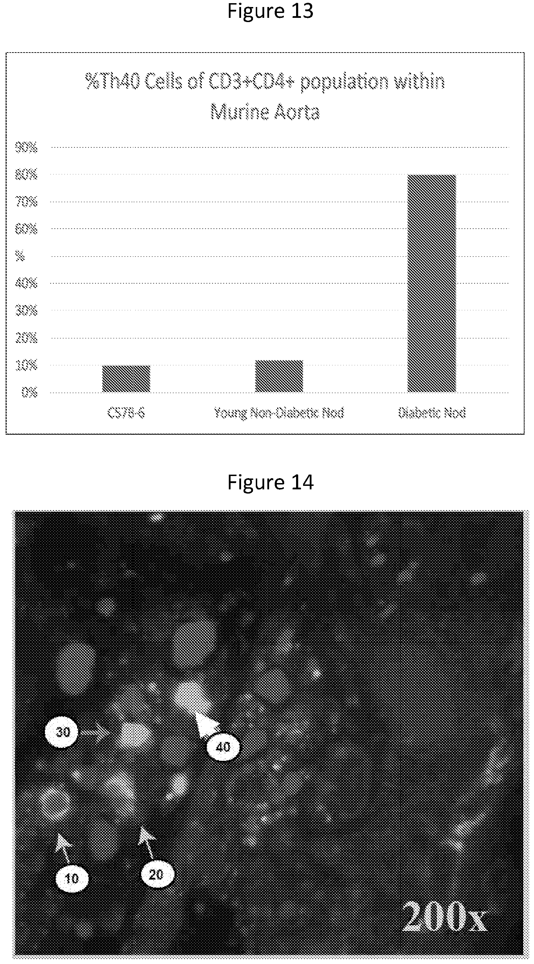

Further, NOD mice tested in this study demonstrated significant Th40 infiltration in the aorta compared with control and young non-diabetic NOD mice populations, as shown in Figure 13.

An exemplar aortic plaque of one of the longitudinal sections was observed at 200x magnification using oil-red-0, trichrome stain and immune-fluorescence, and is shown in Figure 14. This microscopy and stain of the aorta showed that not only are the Th40 cells increased in the aorta similarly to the peripheral blood as demonstrated in Figs. 10-14, but also the Th40 cells are found within the shoulder region of plaque in the ApoE-/-model (the growth region of plaque/atherosclerosis). In Figure 14, **10** and **20** identify cells that represent CD3+, CD4+, and CD40+ (Th40 cells) that have significant intracellular CD40. **30** demonstrates Th40 cell with extracellular expression and no demonstrative CD40 intracellularly. **40** identifies CD3+, CD4+, CD40neg cell.

### Example 8 (not part of the invention)

This example demonstrates interferon gamma production in CD3+CD4+CD40+ cells appear to produce interferon gamma (IFNγ) in abundance. Additionally, interferon gamma controls Th40 proliferation.

ApoE deficient mice on a normal chow diet were selected to receive a dose of 1mg/kg of the 15-mer peptide (SEQ ID NO: 7) by IV tail injection, three times a week over a period of 26 weeks, beginning at 9 weeks of age and also utilized a control. At 25 weeks, the animals were euthanized, weighed, and then had blood, spleen, and pancreas removed for analysis. The subjects were then perfused through cardiac puncture with 4% paraformaldehyde. Aortic arches were dissected, dehydrated in sucrose gradient and then flash frozen. Approximately thirty-five 8µm longitudinal sections were obtained per mouse for various staining procedures. Flow cytometry was performed utilizing a MACSQuant^{®} Analyzer 10 (Miltenyi Biotec Inc.). Additional analysis was performed using FlowJo ^{®} (FlowJo, LLC wholly owned by BectonDickinson, Inc.) single-cell flow cytometry software.

As demonstrated in Figure 14 through confocal microscopy, CD40 can be internal or external to the CD3+CD4+ cell. Flow cytometry was further performed and demonstrated that while most CD3+ cells appear to have ability to produce CD40, the CD3+CD4+CD40+ cells appear to produce interferon gamma (IFNγ) in abundance. This flow cytometry study incorporated both the external and internal staining of CD3, CD4, CD40, and IFNγ.

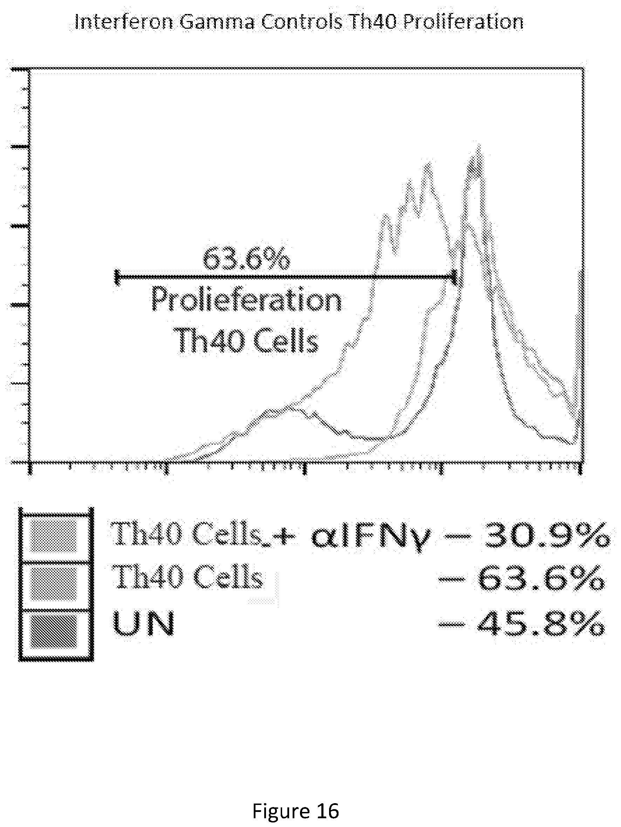

Figure 16 demonstrates that interferon gamma controls Th40 proliferation. Isolated Th40 cells were cross-linked by antibody to CD40. The graph in Figure 16 denotes proliferation of cross-linked (activated) Th40 cells (CD40 XL) vs. antibody to IFNγ and non-cross linked controls (UN). Additionally, by blocking IFNγ, activated Th40 cells do not proliferate.

### Example 9

This example demonstrates that KGYY₁₅ (15-mer - SEQ ID NO:7) abrogates atherosclerosis.

ApoE deficient mice on a normal chow diet were selected to receive a dose of 1mg/kg of the 15-mer peptide (SEQ ID NO: 7) by IV tail injection, three times a week over a period of 26 weeks, beginning at 9 weeks of age and also utilized a control. At 25 weeks, the animals were euthanized, weighed, and then had blood, spleen, and pancreas removed for analysis. The subjects were then perfused through cardiac puncture with 4% paraformaldehyde. Aortic arches were dissected, dehydrated in sucrose gradient and then flash frozen.

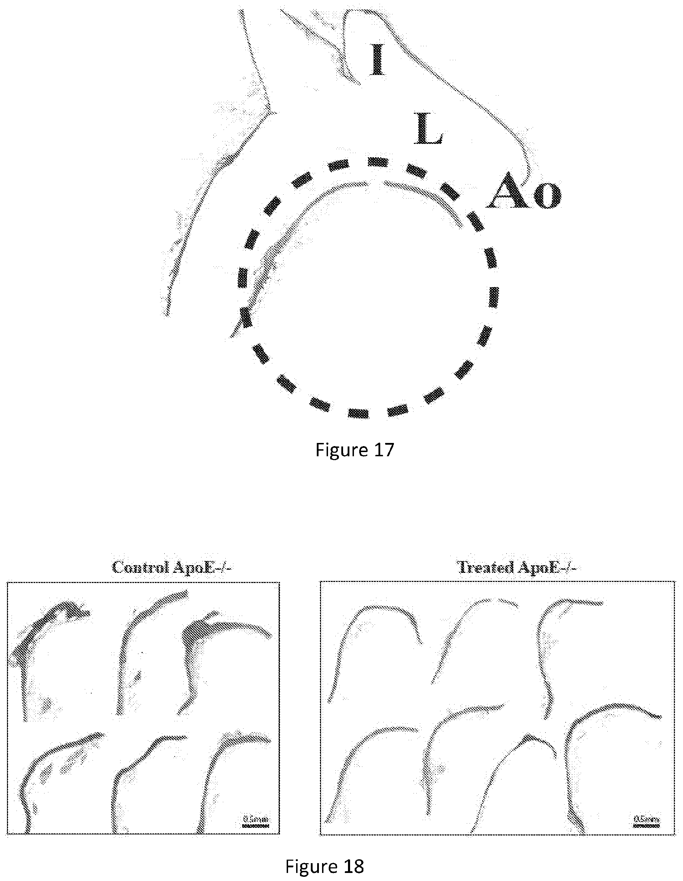

Figure 17 provides an example of the lesser curvature of the aortic arch, defined proximally from the aortic outflow (AO). Of the segments seen in the trichrome stain, an intimal distance of 2.4 mm was measured distally. The aortic-arch wall area subtended by this 2.5 mm stretch of the intima was calculated for each section of all mice, with maximal area of the inner-aortic-arch wall of each mouse used to compute averages per group. The luminal surface (L), aortic arch (AO), and innominate artery (I) are labelled in this Figure 17.

Figure 18 demonstrates the lesser curvature of the aortic arch of the control ApoE mice compared to the lesser curvature of the aortic arch of mice treated with the 15-mer peptide, in accordance with the steps outlined in this example.

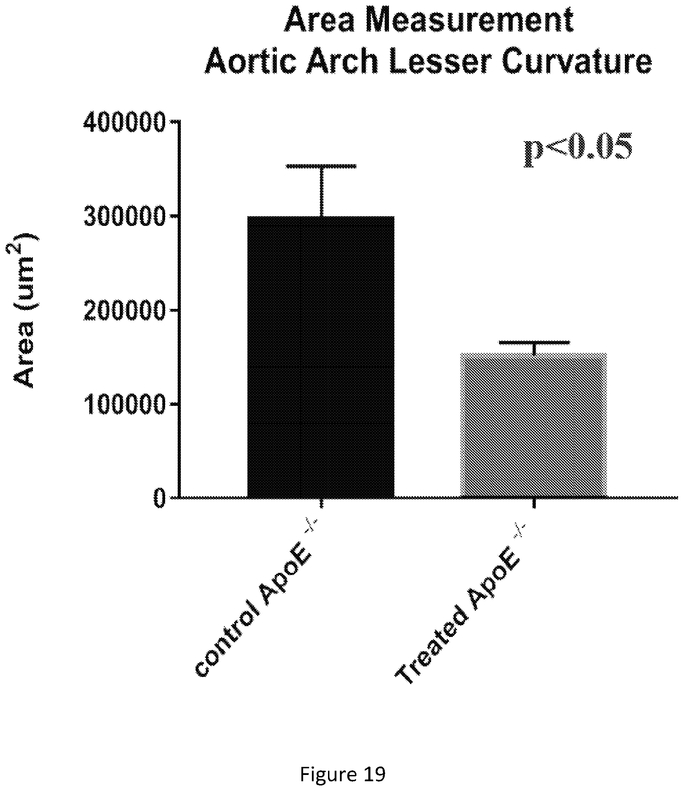

Figure 19 demonstrates the reduction of the total area achieved by peptide treatment. The total area of the 2.5 mm segment (as described in Figure 17) was substantially reduced.

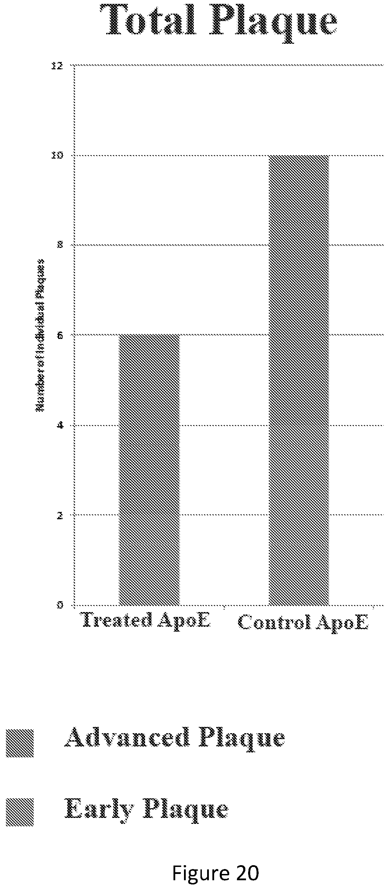

Figure 20 demonstrates the reduction of number of plaque, including early lesions and advanced plaque. The total number of plaque was significantly decreased in the treatment group. Plaque was subdivided based on morphology of early lesions (observable as fatty streaks containing macrophage derived foam cells with varying degrees of lipid accumulation) compared with more advanced fibroatheromas (containing varying degrees of lipid or necrotic core and fibrous caps). All plaque within the designated 2.5mm segment were included. Both the total number and type of plaque were significantly decreased in those subjects treated with the peptide.

This study demonstrates that administration of the peptide abrogates atherosclerosis.

### Example 10

This example demonstrates administration of the KGYY₁₅ (15-mer - SEQ ID NO:7) augments cap formation while reducing advancement of existing disease.

In this study, six ApoE-/- mice received a normal chow diet from 0 to 20 weeks of age. At 20 weeks of age, three mice were randomly assigned to receive dose of 1mg/kg of KGYY₁₅ (15-mer - SEQ ID NO:7) by IV tail injection, once a week for a period of 4 weeks. Control mice received vehicle only. After 4 weeks of treatment, animals were euthanized then perfused through cardiac puncture with 4% paraformaldehyde. Aortic arches were dissected in surcrose gradient and flash frozen. Approximately fifty, 8um longitudinal sections were obtained per mouse. Slides were treated with trichrome stain and analyzed using cellSens software for measurements. Total plaque was measured including 2.5mm lesser curvature and innominate artery.

Figure 20 shows the number of individual early plaques and advanced plaques were reduced in the treated subjects compared to the control subjects.

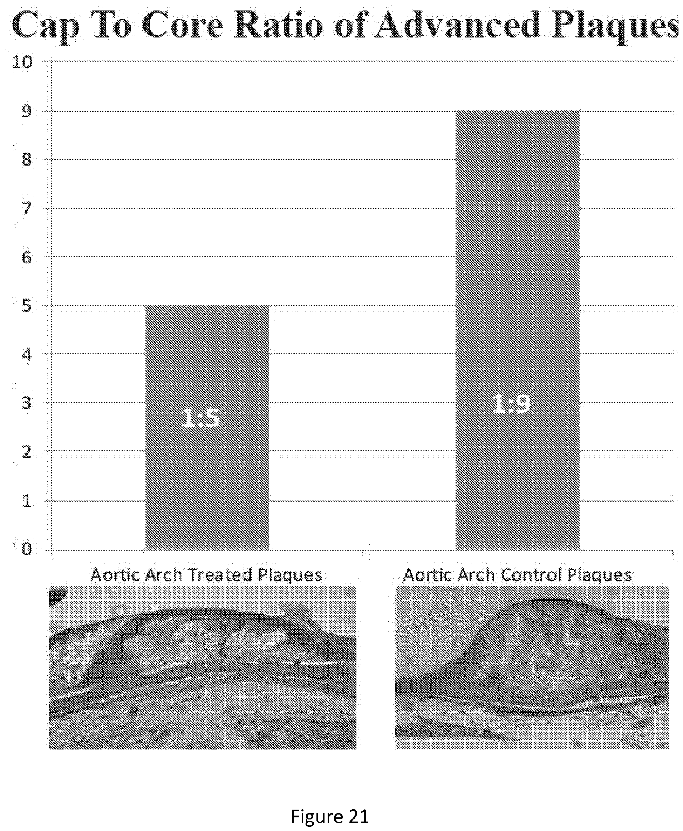

Figure 21 shows that the cap to core ratio of advanced plaques was reduced in the treated subjects compared to the control subjects.

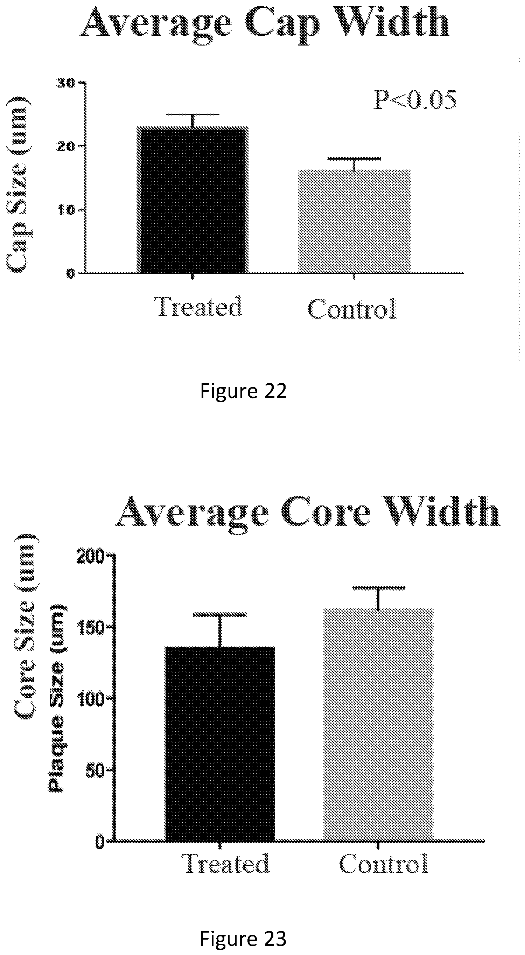

Figure 22 shows the average cap width (cap size) was greater in the treated subjects compared to the control subjects.

Figure 23 shows the average core width (core size/plaque size) was decreased in the subjects treated with the peptide compared to the control subjects.

This example demonstrates that administration of the KGYY₁₅ (15-mer - SEQ ID NO:7) augments cap formation while reducing advancement of existing disease. Moreover, this study further demonstrates that administration of the KGYY₁₅ peptide trends toward results of plaque stability.

From the foregoing, it is readily apparent that T1D shares with atherosclerosis the CD40-CD154 dyad which drives autoimmune inflammation. There are increased Th40 cell levels in peripheral blood of NOD mice, human T1D patients, and ApoE-/- mice. Th40 cells infiltrate the aortic wall and are found within the plaque of ApoE-/- mice. Th40 cells produce the inflammatory cytokine IFNγ at a level greater than that of other cells and this drives inflammation. The KGYY₁₅ peptide targets Th40 cells. The specified peptide furthermore abrogates and modulates atherosclerosis which may be due to Th40 blockade or general blockade of CD40. Moreover, the administration of the specified peptide trends toward more stable plaque types.

### Example 11 (not part of the invention)

Whole human blood was administered the peptide in accordance with similar dosing levels to those used for murine studies.

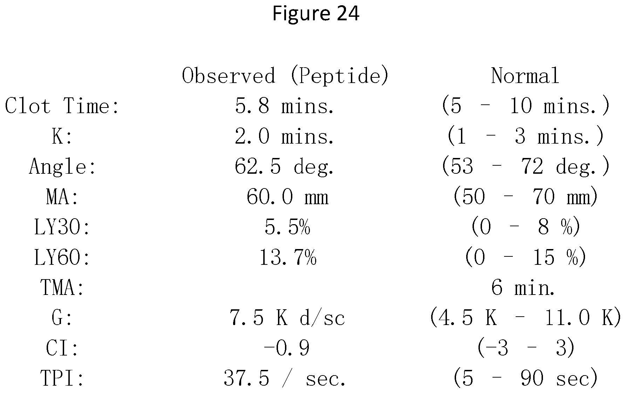

Figure 24 provides the results of clot studies observed in humans.

This study demonstrates that the KGYY15 peptide when administered to humans does not modify or change the clotting of whole human blood significantly outside of normally recognized levels.

### Example 12

This example demonstrates that ApoE mice that have been genetically modified to obtain atherosclerosis and fed a high fat diet and treated with the 6-mer peptide may have the levels of LDL cholesterol values decreased compared to untreated subjects.

In this study, six ApoE-/- mice received a normal chow diet from 0 to 20 weeks of age. At 20 weeks of age, three mice were randomly assigned to receive dose of 1mg/kg of KGYY₆ (6-mer - SEQ ID NO:4) by IV tail injection, once a week for a period of 4 weeks. Control mice received vehicle only.

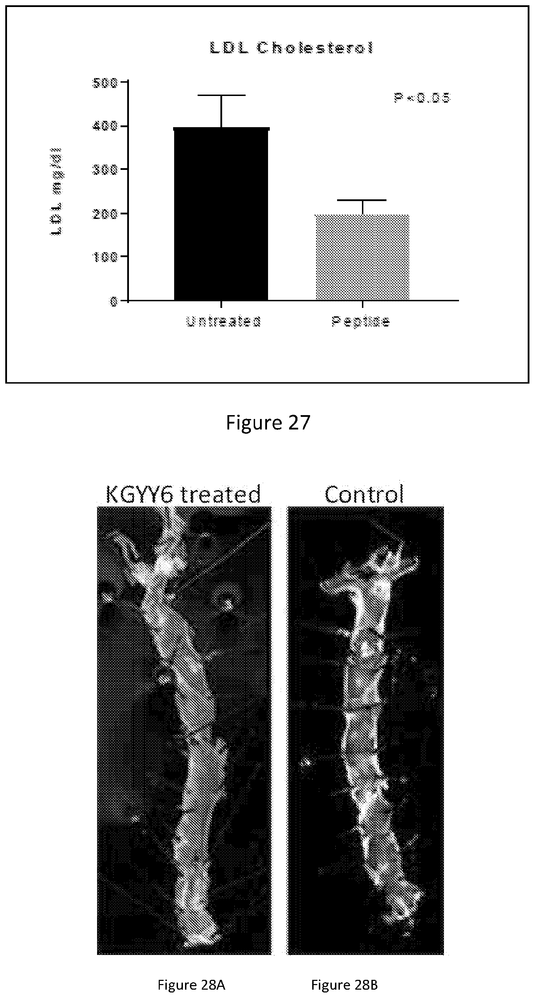

Data obtained from untreated mice and compared to those in treated mice showed statistically significant reduction (>50%) in LDL cholesterol values. This data is provided in Figure 27.

### Example 13

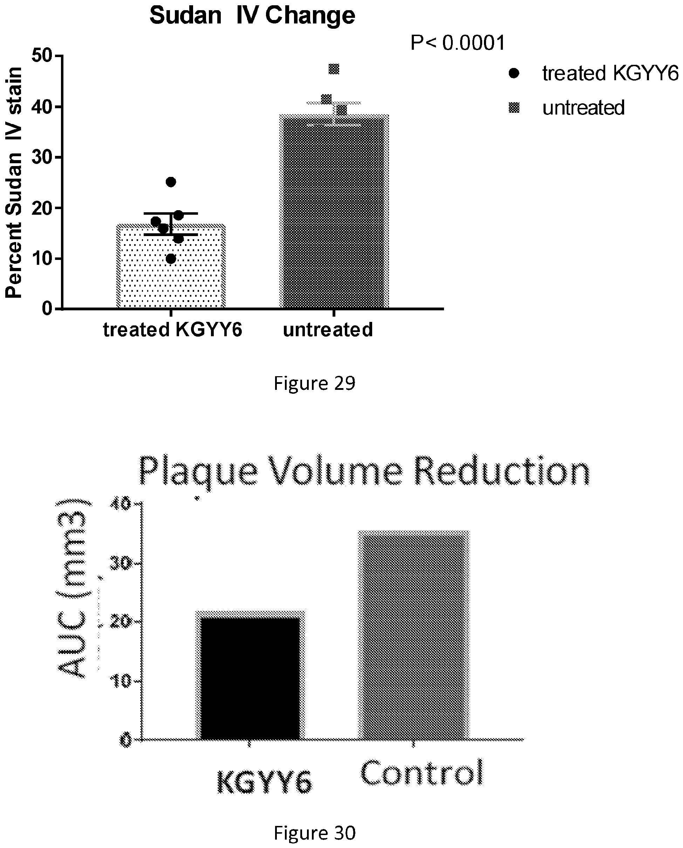

This example demonstrates atherosclerotic changes that ApoE -/- mice experienced when treated with KGYY₆ (6-mer - SEQ ID NO: 4). ApoE -/- mice were fed a high fat diet for 16 weeks. Mice were randomly assigned to receive dose of 1mg/kg of KGYY₆ (6-mer - SEQ ID NO:4) by IV tail injection, once a week for a period of 4 weeks. Control mice received vehicle only. Atherosclerosis was investigated by several methods. En-face analysis utilizing Sudan IV stain (lipid stain) demonstrated a significant reduction in lesion areas. Fig. 28A is an image of KGYY6 treated aortic en-face Sudan IV staining and Fig. 28B is an image of control (untreated) aortic en-face Sudan staining. Fig. 29 is a graph demonstrating the reduction of lesion areas of Sudan IV staining.

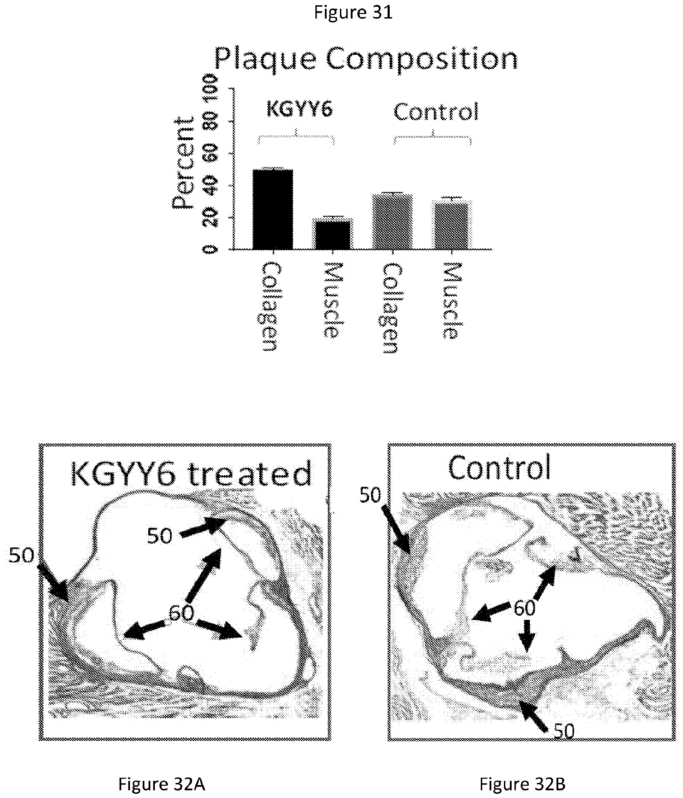

Further, measurement of plaque area and morphology was performed using the Paigen method. This method obtains sequential 5µm aortic cross sections from the aortic root beginning at the valve leaflets into the ascending aorta. At 50µm intervals, slides are stained after which the area of atherosclerotic lesion is measured. Individual plaque area measurements are plotted against the micrometer intervals and a curve is established, with the area under the curve (AUC) giving the total volume of plaque. These results are presented in graph format in Fig. 30, which demonstrates that mice treated with KGYY₆ showed reduction in plaque volume under the curve. Moreover, characterization of plaque composition or aortic samples was performed using trichrome staining techniques and these results are presented in graph format in Fig. 31. Indeed, this Fig. 31 data, which was generated using Image Pro Plus software analysis, quantifies and shows that plaque compositional changes occurred, including increased collagen and reduced smooth muscle content.

Fig. 32A is an image of trichrome stained cells of the cross-sections of the aorta of the KGYY₆ treated subject. Fig. 32B is an image of trichrome stained cells of the cross-sections of the control subjects. In Fig. 32A, **50** identifies areas characteristic of plaque formation. **60** identifies aortic valve leaflets. Fig. 32B, the control (untreated) **50** areas characteristic of plaque (area under the curve) are greater than those in the subjects treated with the KGYY₆ peptide.

From the foregoing, it is readily apparent that new and useful implementations of the methods have been herein described and illustrated which fulfill numerous desiderata in remarkably unexpected fashions. It is, of course, understood that such modifications, alterations and adaptations as may readily occur to the artisan confronted with this disclosure are intended within the spirit of this disclosure, which is limited only by the scope of the claims appended hereto.

## Claims

1. A peptide for use in a method to lower LDL cholesterol, wherein the peptide specifically binds to a CD40 presenting cell at the CD154 binding site, wherein the method comprises administering to a subject in need thereof a therapeutically effective amount of the peptide and wherein said peptide comprises an amino acid sequence selected from SEQ ID NO:3, SEQ ID NO:4, SEQ ID NO:5, SEQ ID NO:6, SEQ ID NO:8, and SEQ ID NO:9.

2. The peptide for use according to claim 1, wherein the peptide comprises an amino acid sequence selected from SEQ ID NO:4 and SEQ ID NO:6.

3. The peptide for use according to claim 1, wherein the peptide affects the interaction of CD40 and CD154.

4. The peptide for use according to claim 1, wherein the peptide inhibits the binding of CD40 to CD154.

5. The peptide for use according to claim 1, wherein the peptide binds CD40 at the site where CD40 interacts with CD154.

6. The peptide for use according to claim 3, wherein the peptide affects the cytokine expression profile of a cell population treated with said peptide.

7. The peptide for use according to claim 2, wherein the peptide is less than 20 amino acids in length.

## Patentansprüche

1. Ein Peptid zur Verwendung in einem Verfahren zur Senkung von LDL-Cholesterin, wobei das Peptid spezifisch an eine CD40-präsentierende Zelle an der CD154-Bindungsstelle bindet, wobei das Verfahren die Verabreichung einer therapeutisch wirksamen Menge des Peptids an einen Patienten umfasst, der dies benötigt, und wobei das Peptid eine Aminosäuresequenz umfasst, die aus SEQ ID NO:3, SEQ ID NO:4, SEQ ID NO:5, SEQ ID NO:6, SEQ ID NO:8 und SEQ ID NO:9 ausgewählt ist.

2. Das Peptid zur Verwendung nach Anspruch 1, wobei das Peptid eine Aminosäuresequenz umfasst, die aus SEQ ID NO:4 und SEQ **ID** NO:6 ausgewählt ist.

3. Das Peptid zur Verwendung nach Anspruch 1, wobei das Peptid die Wechselwirkung von CD40 und CD154 beeinflusst.

4. Das Peptid zur Verwendung nach Anspruch 1, wobei das Peptid die Bindung von CD40 an CD154 hemmt.

5. Das Peptid zur Verwendung nach Anspruch 1, wobei das Peptid CD40 an der Stelle bindet, an der CD40 mit CD154 wechselwirkt.

6. Das Peptid zur Verwendung nach Anspruch 3, wobei das Peptid das Zytokinexpressionsprofil einer mit dem Peptid behandelten Zellpopulation beeinflusst.

7. Das Peptid zur Verwendung nach Anspruch 2, wobei das Peptid weniger als 20 Aminosäuren lang ist.

## Revendications

1. Un peptide pour l'utilisation dans un procédé pour abaisser le cholestérol LDL, dans lequel le peptide se lie spécifiquement à une cellule présentatrice de CD40 au niveau du site de liaison de CD154, dans lequel le procédé comprend l'administration d'une quantité thérapeutiquement efficace du peptide à un patient en ayant besoin et dans lequel ledit peptide comprend une séquence d'acides aminés choisie parmi la SEQ ID NO : 3, la SEQ ID NO : 4, la SEQ ID NO : 5, la SEQ ID NO : 6, la SEQ ID NO : 8 et la SEQ ID NO : 9.

2. Le peptide pour l'utilisation selon la revendication 1, dans lequel le peptide comprend une séquence d'acides aminés choisie parmi la SEQ ID NO : 4 et la SEQ ID NO : 6.

3. Le peptide pour l'utilisation selon la revendication 1, dans lequel le peptide affecte l'interaction de CD40 et CD154.

4. Le peptide pour l'utilisation selon la revendication 1, dans lequel le peptide inhibe la liaison de CD40 à CD154.

5. Le peptide pour l'utilisation selon la revendication 1, dans lequel le peptide se lie à CD40 au niveau du site où CD40 interagit avec CD154.

6. Le peptide pour l'utilisation selon la revendication 3, dans lequel le peptide affecte le profil d'expression des cytokines d'une population cellulaire traitée avec ledit peptide.

7. Le peptide pour l'utilisation selon la revendication 2, dans lequel le peptide a une longueur inférieure à 20 acides aminés.
